(19) Europäisches Patentamt
European Patent Office
Office européen des brevets

(11) EP 3 653 283 A1

(12) EUROPEAN PATENT APPLICATION

(43) Date of publication:
**20.05.2020 Bulletin 2020/21**

(51) Int Cl.:
***B01D 61/14*** *(2006.01)*

(21) Application number: **18206469.1**

(22) Date of filing: **15.11.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BASF SE**
**67056 Ludwigshafen am Rhein (DE)**

(72) Inventors:
- **ARNOLD, Lena**
  **2040 Antwerpen (BE)**
- **MÜLLER, Ulrich**
  **67056 Ludwigshafen (DE)**
- **WENGELER, Lukas**
  **67056 Ludwigshafen (DE)**
- **SCHMIDT, Patrick**
  **51373 Leverkusen (DE)**
- **KARWACKI, Lukasz**
  **67056 Ludwigshafen (DE)**

(74) Representative: **Büchel, Edwin**
**Patentanwälte**
**Isenbruck Bösl Hörschler PartG mbB**
**Eastsite One**
**Seckenheimer Landstraße 4**
**68163 Mannheim (DE)**

# (54) PROCESS FOR CONDITIONING METAL-ORGANIC FRAMEWORKS BY MEANS OF MEMBRANE FILTRATION

(57) The present invention relates to a process for conditioning a raw suspension comprising at least one metal-organic framework and at least one suspension medium by means of at least one membrane filtration to obtain a product suspension. The invention relates also to a method, wherein said product suspension is coated to at least part of the surface of a substrate.

EP 3 653 283 A1

## Description

The present invention relates to a process for conditioning a raw suspension comprising at least one metal-organic framework and at least one suspension medium by means of at least one membrane filtration to obtain a product suspension. The invention relates also to a method, wherein the product suspension is coated to at least part of the surface of a substrate.

Porous materials useful in various adsorption applications are known. As an example zeolites are described by M. Tatlıer et al., Microporous and Mesoporous Materials 34 (2000), 23-30, for the optimization of the cycle duration of adsorption heat pumps.

Porous metal-organic frameworks are known in the prior art and form an interesting class of substances, which can be an alternative to zeolites, activated carbons and other porous materials for various applications such as heterogeneous catalysis.

In applications, such as adsorption heat pumps or air conditioning systems, a metal-organic framework, for example Aluminum fumarate (A520) needs to be applied as functional coating, which is then coated on different substrates.

Current processes for preparing metal organic frameworks and coating compositions comprising metal-organic frameworks comprises the following steps: First, the MOF is prepared by reacting a metal ion and a bidentate organic compound in presence of a solvent wherein a MOF-containing suspension is obtained. Afterwards the MOF-containing suspension is typically isolated by means of conventional filtration, washing and subsequent drying, typically spray-drying. Afterwards, the coating composition is formulated by adding a liquid as suspension medium and typically at least one binder compound until the desired MOF concentration is obtained.

The conventional method as described above is accompanied by several disadvantages; especially it is energy- and material-intensive and produces high amounts of waste-water, and results therefore in relatively high process costs.

Furthermore, it could be observed, that MOF-films formed on the surface of a substrate by means of conventional coating compositions, i.e. compositions formulated from MOF-powder, a liquid medium and usually a binder, often has several undesired properties regarding crack formation, adhesion properties and density.

There is therefore a need for a process for conditioning a MOF-containing raw suspension, which is more energy- and cost-efficient than known processes.

Moreover, there is a need for a method for coating a substrate with a MOF-film, wherein the MOF-film shows improved properties in view of conventionally produced MOF-films.
It is therefore one object of the present invention to provide such a process for conditioning.

This objective is achieved by a process for conditioning a raw suspension SR comprising at least one metal-organic framework and at least one suspension medium SM1,
wherein the at least one metal-organic framework comprises at least one at least bidentate organic compound coordinated to at least one metal ion,
wherein the raw suspension SR is conditioned by means of at least one membrane filtration to obtain a product suspension SP, comprising the at least one metal-organic framework and at least one suspension medium SM2.

Surprisingly, it has been found that the inventive process is less energy-consuming than conventional techniques and provides a product suspension SP, which can be directly used, for example, for coating a MOF-film on the surface of a substrate. Moreover, it has been found that such MOF-films show improved properties, in particular a higher density and less crack formation of the film could be observed.

Metal-organic Framework

The metal-organic framework according to the present invention comprises pores, in particular micropores and/or mesopores. Micropores are defined as pores having a diameter of 2 nm or less and mesopores are defined by a diameter in the range from 2 to 50 nm, in each case in accordance with the definition given in Pure & Applied Chem. 57 (1983), 603-619, in particular on page 606. The presence of micropores and/or mesopores can be checked by means of sorption measurements, with these measurements determining the uptake capacity of the MOFs for nitrogen at 77 Kelvin (Langmuir Method) in accordance with DIN 66131:1993-07 and/or DIN 66134:1998-2 or according to the BET-Method (DIN ISO 9277:2003-05).

The specific surface area, determined according to BET (DIN ISO 9277:2003-05) by $N_2$ adsorption, of a metal organic framework in powder form is preferably more than 100 $m^2/g$, more preferably above 300 $m^2/g$, more preferably more than 500 $m^2/g$, even more preferably more than 800 $m^2/g$, even more preferably more than 1000 $m^2/g$ and particularly preferably more than 1200 $m^2/g$.

The metal-organic framework according to the invention comprises at least one metal-ion.

The metal ion in the framework according to the present invention is preferably an ion of a metal selected from groups Ia, IIa, IIIa, IVa to Villa and Ib to Vlb. Particular preference is given to ions of Mg, Ca, Sr, Ba, Sc, Y, Ln, Ti, Zr,

Hf, V, Nb, Ta, Cr, Mo, W, Mn, Re, Fe, Ro, Os, Co, Rh, Ir, Ni, Pd, Pt, Cu, Ag, Au, Zn, Cd, Hg, Al, Ga, In, Tl, Si, Ge, Sn, Pb, As, Sb and Bi, wherein Ln represents lanthanides.

Lanthanides are La, Ce, Pr, Nd, Pm, Sm, En, Gd, Tb, Dy, Ho, Er, Tm, Yb.

As regards the ions of these elements, particular mention may be made of $Mg^{2+}$, $Ca^{2+}$, $Sr^{2+}$, $Ba^{2+}$, $Sc^{3+}$, $Y^{3+}$, $Ln^{3+}$, $Ti^{4+}$, $Zr^{4+}$, $Hf^{4+}$, $V^{4+}$, $V^{3+}$, $V^{2+}$, $Nb^{3+}$, $Ta^{3+}$, $Cr^{3+}$, $Mo^{3+}$, $W^{3+}$, $Mn^{3+}$, $Mn^{2+}$, $Re^{3+}$, $Re^{2+}$, $Fe^{3+}$, $Fe^{2+}$, $Ru^{3+}$, $Ru^{2+}$, $Os^{3+}$, $Os^{2+}$, $Co^{3+}$, $Co^{2+}$, $Rh^{2+}$, $Rh^{+}$, $Ir^{2+}$, $Ir^{+}$, $Ni^{2+}$, $Ni^{+}$, $Pd^{2+}$, $Pd^{+}$, $Pt^{2+}$, $Pt^{+}$, $Cu^{2+}$, $Cu^{+}$, $Ag^{+}$, $Au^{+}$, $Zn^{2+}$, $Cd^{2+}$, $Hg^{2+}$, $Al^{3+}$, $Ga^{3+}$, $In^{3+}$, $Tl^{3+}$, $Si^{4+}$, $Si^{2+}$, $Ge^{4+}$, $Ge^{2+}$, $Sn^{4+}$, $Sn^{2+}$, $Pb^{4+}$, $Pb^{2+}$, $As^{5+}$, $As^{3+}$, $As^{+}$, $Sb^{5+}$, $Sb^{3+}$, $Sb^{+}$, $Bi^{5+}$, $Bi^{3+}$ and $Bi^{+}$.

Preference is given to the ions of Zn, Al, Mg, Cu, Mn, Fe, Co, Ni, Ti, Zr, Y, Sc, V, In, Ca, Cr, Mo, W, Ln. Preferably the at least one metal ion is an ion of Cu, Zn, Al, Mg, Zr, and Fe. Particular preference is given to the ions of Cu, Zn, Mg and Al, preferably $Cu^{2+}$, $Zn^{2+}$ , $Mg^{2+}$, and $Al^{3+}$, very particular preference is given to $Al^{3+}$.

In the process according to the invention the metal ion is used in the form of at least one metal salt corresponding to the at least one metal ion.

Preferably at least one anion of the at least one metal salt is oxide, hydroxide, acetate, chloride, carbonate, sulfate, nitrate, or a mixture of two or more thereof, preferably hydroxide, oxide, carbonate, sulfate or a mixture of two or more thereof.

In a preferred embodiment the metal salt is copper hydroxide ($Cu(OH)_2$), zink carbonate ($ZnCO_3$), basic zinc carbonate $[Zn(CO_3)]_2[Zn(OH)_2]_3$, aluminum sulfate $Al_2(SO_4)_3$, or hydrates thereof, or any metal hydroxide, particularly preferred are ($Cu(OH)_2$), basic zinc carbonate $[Zn(CO_3)]_2[Zn(OH)_2]_3$, aluminum sulfate $Al_2(SO_4)_3$, or hydrates thereof.

The term "at least bidentate organic compound" refers to an organic compound which comprises at least one functional group which is able to form at least two coordinate bonds to a given metal ion and/or form a coordinate bond to each of two or more, preferably two, metal atoms. The at least one at least bidentate organic compound can be used as such or in the form of a salt thereof, preferably the organic compound is used as such.

As functional groups via which the abovementioned coordinate bonds can be formed, mention may be made by way of example of, in particular: $-CO_2H$, $-CS_2H$, $-NO_2$, $-B(OH)_2$, $-SO_3H$,$-Si(OH)_3$, $-Ge(OH)_3$, $-Sn(OH)_3$, $-Si(SH)_4$, $-Ge(SH)_4$, $-Sn(SH)_3$, $-PO_3H$, $-AsO_3H$, $-AsO_4H$, $-P(SH)_3$, $-As(SH)_3$, $-CH(RSH)_2$, $-C(RSH)_3$ $-CH(RNH_2)_2$ $-C(RNH_2)_3$, $-CH(ROH)_2$, $-C(ROH)_3$, $-CH(RCN)_2$,$-C(RCN)_3$, where R is preferably, for example, an alkylene group having 1, 2, 3, 4 or 5 carbon atoms, for example a methylene, ethylene, n-propylene, i-propylene, n-butylene, i-butylene, tert-butylene or n-pentylene group, or an aryl group comprising 1 or 2 aromatic rings, for example 2 $C_6$ rings, which may, if appropriate, be fused and may, independently of one another, be appropriately substituted by in each case at least one substituent and/or may, independently of one another, comprise in each case at least one heteroatom, for example N, O and/or S. In likewise preferred embodiments, mention may be made of functional groups in which the abovementioned radical R is not present. In this regard, mention may be made of, inter alia, $-CH(SH)_2$,$-C(SH)_3$, $-CH(NH_2)_2$, $-C(NH_2)_3$, $-CH(OH)_2$, $-C(OH)_3$, $-CH(CN)_2$ or $-C(CN)_3$.

However, the functional groups can also be heteroatoms of a heterocycle. Particular mention may here be made of nitrogen atoms.

The at least two functional groups can in principle be bound to any suitable organic compound as long as it is ensured that the organic compound comprising these functional groups is capable of forming the coordinate bond and of producing the framework.

The organic compounds which comprise at least two functional groups are preferably derived from a saturated or unsaturated aliphatic compound or an aromatic compound or a both aliphatic and aromatic compound.

The aliphatic compound or the aliphatic part of the both aliphatic and aromatic compound can be linear and/or branched and/or cyclic, with a plurality of rings per compound also being possible. The aliphatic compound or the aliphatic part of the both aliphatic and aromatic compound more preferably comprises from 1 to 15, more preferably from 1 to 14, more preferably from 1 to 13, more preferably from 1 to 12, more preferably from 1 to 11 and particularly preferably from 1 to 10, carbon atoms, for example 1, 2, 3, 4, 5, 6, 7, 8, 9 or 10 carbon atoms. Particular preference is here given to, inter alia, methane, adamantane, acetylene, ethylene or butadiene.

The aromatic compound or the aromatic part of the both aromatic and aliphatic compound can have one or more rings, for example two, three, four or five rings, with the rings being able to be present separately from one another and/or at least two rings being able to be present in fused form. The aromatic compound or the aromatic part of the both aliphatic and aromatic compound particularly preferably has one, two or three rings, with particular preference being given to one or two rings. Furthermore, the rings of said compound can each comprise, independently of one another, at least one heteroatom such as N, O, S, B, P, Si, Al, preferably N, O and/or S. More preferably, the aromatic compound or the aromatic part of the both aromatic and aliphatic compound comprises one or two $C_6$ rings; in the case of two rings, they can be present either separately from one another or in fused form. Aromatic compounds of which particular mention may be made are benzene, naphthalene and/or biphenyl and/or bipyridyl and/or pyridyl.

The at least bidentate organic compound is more preferably an aliphatic or aromatic, acyclic or cyclic hydrocarbon which has from 1 to 18, preferably from 1 to 10 and in particular 6, carbon atoms and in addition has exclusively 2, 3 or 4 carboxyl groups as functional groups.

For example, the at least bidentate organic compound is derived from a dicarboxylic acid such as oxalic acid, succinic acid, tartaric acid, 1,4-butanedicarboxylic acid, 1,4-butenedicarboxylic acid, 4-oxopyran-2,6-dicarboxylic acid, 1,6-hexanedicarboxylic acid, decanedicarboxylic acid, 1,8-heptadecanedicarboxylic acid, 1,9-heptadecanedicarboxylic acid, heptadecanedicarboxylic acid, acetylenedicarboxylic acid, 1,2-benzenedicarboxylic acid, 1,3-benzenedicarboxylic acid, 2,3-pyridinedicarboxylic acid, pyridine-2,3-dicarboxylic acid, 1,3-butadiene-1,4-dicarboxylic acid, 1,4-benzenedicarboxylic acid, p-benzenedicarboxylic acid, imidazole-2,4-dicarboxylic acid, 2-methylquinoline-3,4-dicarboxylic acid, quinoline-2,4-dicarboxylic acid, quinoxaline-2,3-dicarboxylic acid, 6-chloroquinoxaline-2,3-dicarboxylic acid, 4,4'-diaminophenylmethane-3,3'-dicarboxylic acid, quinoline-3,4-dicarboxylic acid, 7-chloro-4-hydroxyquinoline-2,8-dicarboxylic acid, diimidedicarboxylic acid, pyridine-2,6-dicarboxylic acid, 2-methylimidazole-4,5-dicarboxylic acid, thiophene-3,4-dicarboxylic acid, 2-isopropylimidazole-4,5-dicarboxylic acid, tetrahydropyran-4,4-dicarboxylic acid, perylene-3,9-dicarboxylic acid, perylenedicarboxylic acid, Pluriol E 200-dicarboxylic acid, 3,6-dioxaoctanedicarboxylic acid, 3,5-cyclohexadiene-1,2-dicarboxylic acid, octadicarboxylic acid, pentane-3,3-dicarboxylic acid, 4,4'-diamino-1,1'-biphenyl-3,3'-dicarboxylic acid, 4,4'-diaminobiphenyl-3,3'-dicarboxylic acid, benzidine-3,3'-dicarboxylic acid, 1,4-bis(phenylamino)benzene-2,5-dicarboxylic acid, 1,1'-binaphthyldicarboxylic acid, 7-chloro-8-methylquinoline-2,3-dicarboxylic acid, 1-anilinoanthraquinone-2,4'-dicarboxylic acid, polytetra-hydrofuran-250-dicarboxylic acid, 1,4-bis(carboxymethyl)piperazine-2,3-dicarboxylic acid, 7-chloroquinoline-3,8-dicarboxylic acid, 1-(4-carboxy)phenyl-3-(4-chloro)phenylpyrazoline-4,5-dicarboxylic acid, 1,4,5,6,7,7-hexachloro-5-norbornene-2,3-dicarboxylic acid, phenylindane-dicarboxylic acid, 1,3-dibenzyl-2-oxoimidazolidine-4,5-dicarboxylic acid, 1,4-cyclohexanedicarboxylic acid, naphthalene-1,8-dicarboxylic acid, 2-benzoylbenzene-1,3-dicarboxylic acid, 1,3-dibenzyl-2-oxoimidazolidine-4,5-cis-dicarboxylic acid, 2,2'-biquinoline-4,4'-dicarboxylic acid, pyridine-3,4-dicarboxylic acid, 3,6,9-trioxaundecanedicarboxylic acid, hydroxybenzophenonedi-carboxylic acid, Pluriol E 300-dicarboxylic acid, Pluriol E 400-dicarboxylic acid, Pluriol E 600-dicarboxylic acid, pyrazole-3,4-dicarboxylic acid, 2,3-pyrazinedicarboxylic acid, 5,6-dimethyl-2,3-pyrazinedicarboxylic acid, (bis(4-aminophenyl)ether)diimidedicarboxylic acid, 4,4'-diamino-diphenylmethanediimidedicarboxylic acid, (bis(4-aminophenyl) sulfone)diimidedicarboxylic acid, 1,4-naphthalenedicarboxylic acid, 2,6-naphthalenedicarboxylic acid, 1,3-adamantanedicarboxylic acid, 1,8-naphthalenedicarboxylic acid, 2,3-naphthalenedicarboxylic acid, 8-methoxy-2,3-naphthalenedicarboxylic acid, 8-nitro-2,3-naphthalenecarboxylic acid, 8-sulfo-2,3-naphthalenedicarboxylic acid, anthracene-2,3-dicarboxylic acid, 2',3'-diphenyl-p-terphenyl-4,4"-dicarboxylic acid, (diphenyl ether)-4,4'-dicarboxylic acid, imidazole-4,5-dicarboxylic acid, 4(1H)-oxothiochromene-2,8-dicarboxylic acid, 5-tert-butyl-1,3-benzenedicarboxylic acid, 7,8-quinolinedicarboxylic acid, 4,5-imidazoledicarboxylic acid, 4-cyclohexene-1,2-dicarboxylic acid, hexatri-acontanedicarboxylic acid, tetradecanedicarboxylic acid, 1,7-heptadicarboxylic acid, 5-hydroxy-1,3-benzenedicarboxylic acid, 2,5-dihydroxy-1,4-dicarboxylic acid, pyrazine-2,3-dicarboxylic acid, furan-2,5-dicarboxylic acid, 1-nonene-6,9-dicarboxylic acid, eicosenedicarboxylic acid, 4,4'-dihydroxydiphenylmethane-3,3'-dicarboxylic acid, 1-amino-4-methyl-9,10-dioxo-9,10-dihydroanthracene-2,3-dicarboxylic acid, 2,5-pyridinedicarboxylic acid, cyclohexene-2,3-dicarboxylic acid, 2,9-dichlorofluorubin-4,11-dicarboxylic acid, 7-chloro-3-methylquinoline-6,8-dicarboxylic acid, 2,4-dichlorobenzophenone-2',5'-dicarboxylic acid, 1,3-benzenedicarboxylic acid, 2,6-pyridinedicarboxylic acid, 1-methylpyrrole-3,4-dicarboxylic acid, 1-benzyl-1H-pyrrole-3,4-dicarboxylic acid, anthraquinone-1,5-dicarboxylic acid, 3,5-pyrazoledicarboxylic acid, 2-nitrobenzene-1,4-dicarboxylic acid, heptane-1,7-dicarboxylic acid, cyclobutane-1,1-dicarboxylic acid, 1,14-tetradecanedicarboxylic acid, 5,6-dehydronorbornane-2,3-dicarboxylic acid, 5-ethyl-2,3-pyridinedicarboxylic acid or camphordicarboxylic acid.

The at least bidentate organic compound is even more preferably one of the dicarboxylic acids mentioned above by way of example as such.

For example, the at least bidentate organic compound can be derived from a tricarboxylic acid such as 2-hydroxy-1,2,3-propanetricarboxylic acid, 7-chloro-2,3,8-quinolinetricarboxylic acid, 1,2,3-, 1,2,4-benzenetricarboxylic acid, 1,2,4-butanetricarboxylic acid, 2-phosphono-1,2,4-butanetricarboxylic acid, 1,3,5-benzenetricarboxylic acid, 1-hydroxy-1,2,3-propanetricarboxylic acid, 4,5-dihydro-4,5-dioxo-1H-pyrrolo[2,3-F]quinoline-2,7,9-tricarboxylic acid, 5-acetyl-3-amino-6-methylbenzene-1,2,4-tricarboxylic acid, 3-amino-5-benzoyl-6-methylbenzene-1,2,4-tricarboxylic acid, 1,2,3-propanetricarboxylic acid or aurintricarboxylic acid.

The at least bidentate organic compound is even more preferably derived from one of the tricarboxylic acids mentioned above by way of example as such.

Examples of an at least bidentate organic compound derived from a tetracarboxylic acid are

1,1-dioxidoperylo[1,12-BCD]thiophene-3,4,9,10-tetracarboxylic acid, perylenetetracarboxylic acids such as perylene-3,4,9,10-tetracarboxylic acid or (perylene 1,12-sulfone)-3,4,9,10-tetracarboxylic acid, butanetetracarboxylic acids such as 1,2,3,4-butanetetracarboxylic acid or meso-1,2,3,4-butanetetracarboxylic acid, decane-2,4,6,8-tetracarboxylic acid, 1,4,7,10,13,16-hexa-oxacyclooctadecane-2,3,11,12-tetracarboxylic acid, 1,2,4,5-benzenetetracarboxylic acid, 1,2,11,12-dodecanetetracarboxylic acid, 1,2,5,6-hexanetetracarboxylic acid, 1,2,7,8-octane-tetracarboxylic acid, 1,4,5,8-naphthalenetetracarboxylic acid, 1,2,9,10-decanetetracarboxylic acid, benzophenonetetracarboxylic acid,

3,3',4,4'-benzophenonetetracarboxylic acid, tetra-hydrofurantetracarboxylic acid or cyclopentanetetracarboxylic acids such as cyclopentane-1,2,3,4-tetracarboxylic acid.

The at least bidentate organic compound is even more preferably one of the tetracarboxylic acids mentioned above by way of example as such.

In a preferred embodiment, the at least one at least bidentate organic compound is thus derived from a dicarboxylic, tricarboxylic or tetracarboxylic acid or is such an acid.

Preference is also given to using optionally at least monosubstituted aromatic dicarboxylic, tricarboxylic or tetracarboxylic acids which have one, two, three, four or more rings and in which each of the rings can comprise at least one heteroatom, with two or more rings being able to comprise identical or different heteroatoms. For example, preference is given to one-ring dicarboxylic acids, one-ring tricarboxylic acids, one-ring tetracarboxylic acids, two-ring dicarboxylic acids, two-ring tricarboxylic acids, two-ring tetracarboxylic acids, three-ring dicarboxylic acids, three-ring tricarboxylic acids, three-ring tetracarboxylic acids, four-ring dicarboxylic acids, four-ring tricarboxylic acids and/or four-ring tetracarboxylic acids. Suitable heteroatoms are, for example, N, O, S, B, P and preferred heteroatoms here are N, S and/or O. Suitable substituents which may be mentioned in this respect are, inter alia, -OH, a nitro group, an amino group or an alkyl or alkoxy group.

For the purposes of the present invention, the term "derived" means that the dicarboxylic, tricarboxylic or tetracarboxylic acid can be present in partially deprotonated or fully deprotonated form in the framework. Furthermore, the dicarboxylic, tricarboxylic or tetracarboxylic acid can comprise a substituent or, independently of one another, a plurality of substituents. Examples of such substituents are -OH, $-NH_2$, $-OCH_3$, $-CH_3$, $-NH(CH_3)$, $-N(CH_3)_2$, -CN and halides. Furthermore, the term "derived" means, for the purposes of the present invention, that the dicarboxylic, tricarboxylic or tetracarboxylic acid can also be present in the form of the corresponding sulfur analogues. Sulfur analogues are the functional groups -C(=O)SH and its tautomer and C(=S)SH, which can be used instead of one or more carboxylic acid groups. Furthermore, the term "derived" means, for the purposes of the present invention, that one or more carboxylic acid fractions can be replaced by a sulfonic acid group ($-SO_3H$). Furthermore, it is likewise possible for a sulfonic acid group to be present in addition to the 2, 3 or 4 carboxylic acid functions.

Preferred monocarboxylic acids as at least bidentate compound in which a coordinate bond is formed via the carboxylic functional group are formates and mixed formates/acetates especially in form of Mg- and Li-MOFs (WO 2009/115513 A1 and WO 2010/012715 A1).

In another preferred embodiment the at least one at least bidentate organic compound is a monocyclic, bicyclic or polycyclic ring system which is derived from at least one heterocycle selected from the group consisting of pyrrole, alpha-pyridone and gamma-pyridone. All these three heterocycles have a ring nitrogen which in at least one limiting structure bears a hydrogen atom which can be split off. It is thus possible to deprotonate pyrrole, alpha-pyridone or gamma-pyridone. This forms a negative charge which can at least partly balance the positive charge of the at least one metal ion.

For the purposes of the present invention, the term "derive" in this context means that the monocyclic, bicyclic or polycyclic ring system has at least one substructure which corresponds to pyrrole, alpha-pyridone or gamma-pyridone. Furthermore, two or all three heterocycles can also be present as substructure in the ring system.

For the purposes of the present invention, the term "derive" also means that the three above-mentioned heterocycles can occur not in neutral form but, if appropriate, also as anion or cation.

Furthermore, it should be noted that at least one of the heterocycles which represents a substructure of the ring system may be deprotonated during the reaction.

Furthermore, for the purposes of the present invention, the term "derive" means that the substructure of at least one of the three heterocycles can bear substituents and one or more ring carbons can be replaced by a heteroatom.

Of course, the ring system can also be one of the heterocycles pyrrole, alpha-pyridone or gamma-pyridone itself or the ring system can likewise be made up of substructures which are selected exclusively from the group consisting of pyrrole, alpha-pyridone and gamma-pyridone. In this case too, the above-described modifications are possible.

Finally, it should be noted that at least one hydrogen which in at least one limiting structure is not the hydrogen bound to said nitrogen is replaced by a bond by means of which the respective heterocycle is bound to the remainder of the ring system.

If a monocyclic ring system is present, this is derived from pyrrole or alpha-pyridone or gamma-pyridone.

However, the ring system can also be a bicyclic ring system. This is the case when, for example, two rings which are joined to one another via a covalent single bond or via a group R are present in the ring system. Here, one ring has to be derived from pyrrole, alpha-pyridone or gamma-pyridone.

R can be -O-, -NH-, -S-, -N=N- or an aliphatic branched or unbranched saturated or unsaturated hydrocarbon which has from 1 to 4 carbon atoms and may be interrupted by one or more atoms or functional groups selected independently from the group consisting of -O-, -NH-, -S- and - N=N-.

Furthermore, the bicyclic ring system can be a fused ring system.

Examples are, in particular, benzo-fused derivatives derived from pyrrole, alpha-pyridone and gamma-pyridone.

In addition, the bicyclic ring system can be a bridged ring system.

The ring system can likewise be a polycyclic ring system which has, for example, 3, 4 or more rings. Here, the rings can be joined via a covalent single bond and/or a group R and/or be fused and/or be present as a bridged ring system.

The ring system has at least two ring nitrogens. Here, at least one of the two ring nitrogens is that nitrogen which is present in the ring derived from pyrrole, alpha-pyridone or gamma-pyridone. In addition, at least one further ring nitrogen has to be present. If the ring system is one which has more than one ring, the at least second ring nitrogen can also be present in the ring derived from pyrrole, alpha-pyridone or gamma-pyridone or, if the at least one further ring is not derived from one of these three heterocycles, may be located in this ring.

The at least two ring nitrogens are preferably present in one ring of the ring system.

In this case, the ring is derived from pyrazole, imidazole, pyridazin-2-one or pyrimidin-2-one or pyrimidin-4-one. Preference is given to imidazole.

In addition to the two ring nitrogens, further ring nitrogens can be present. For example, the ring system can have 3, 4, 5 or more ring nitrogens.

If more than two ring nitrogens are present, all ring nitrogens can be present in one ring of the ring system or can be distributed over more than one ring up to all rings of the ring system.

If, for example, three ring nitrogens are present, these are also preferably present in the ring which is derived from pyrrole, alpha-pyridone or gamma-pyridone. The resulting substructure of the ring can then be derived, for example, from a triazole, such as 1,2,3-triazole or 1,2,4-triazole.

In addition, the ring system can have further heteroatoms in the ring. These can be, for example, oxygen or sulfur. However, preference is given to no further heteroatoms in addition to nitrogen being present.

If the ring system has more than one ring, this ring can be saturated or unsaturated. The at least one further ring preferably has an at least partially conjugated double bond system or is aromatic in nature.

The ring system can be unsubstituted.

The ring system can also have one or more substituents. If a plurality of substituents is present, these can be identical or different. Preference is given to substituted imidazoles.

The substituents bound to the ring system can be halogen, $C_{1-6}$-alkyl, phenyl, $NH_2$, $NH(C_{1-6}$-alkyl), $N(C_{1-6}$-alkyl$)_2$, OH, Ophenyl or $OC_{1-6}$-alkyl.

If at least one of the abovementioned substituents of the ring system is a $C_{1-6}$-alkyl or phenyl, these can likewise be unsubstituted or bear one or more substituents. When a plurality of substituents is present, it is also possible here for them to be identical or different. These are selected from the group consisting of halogen, $NH_2$, $NH(C_{1-6}$-alkyl), $N(C_{1-6}$-alkyl), $N(C_{1-6}$-alkyl$)_2$, OH, Ophenyl and $OC_{1-6}$-alkyl.

If the group $C_{1-6}$-alkyl occurs more than once, these alkyl groups can be identical or different.

For the purposes of the present invention, the hydroxy or keto group of alpha-pyridone and gamma-pyridone is not counted as a substituent since this group is necessarily present in the ring in order to obtain, at least for one limiting structure, a ring nitrogen bound to hydrogen.

Preference is given to the substituents bound to the ring system having no further substituents.

Preferred substituents bound to the ring system are $C_{1-6}$-alkyl, phenyl, $NH_2$ and OH. $C_{1-6}$-alkyl and $NH_2$ are more preferred. Particular preference is given to $C_{1-6}$-alkyl.

In a further preferred embodiment, the ring system is selected from the group consisting of

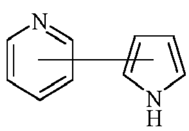 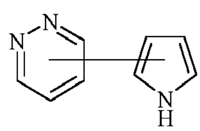 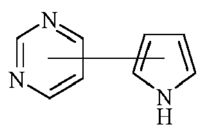 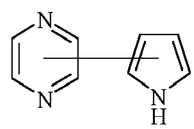

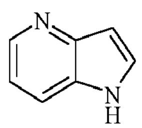 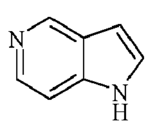 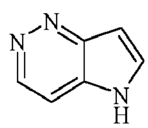 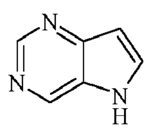 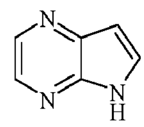

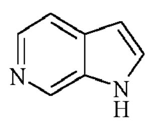 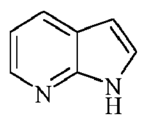 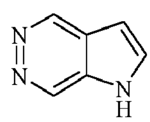 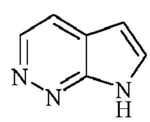 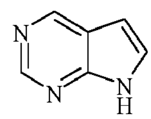

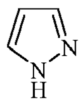 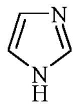 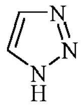 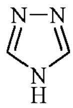 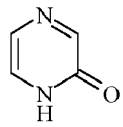 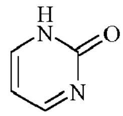 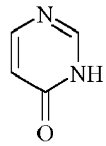

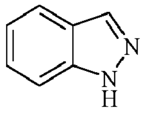 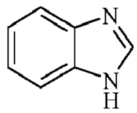 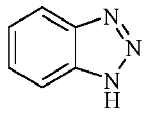 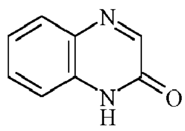

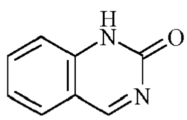 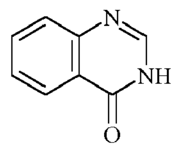

Further preferred ring systems are an imidazole, benzimidazole, triazole, 2-hydroxypyrimidine or 4-hydroxypyrimidine, very particularly preferably selected from the group consisting of 2-methylimidazole, 2-ethylimidazole, benzimidazole, 1,2,4-triazole, 3-amino-1,2,4-triazole, 3,5-diamino-1,2,4-triazole, 2-hydroxypyrimidine and 4-hydroxypyrimidine and their deprotonated forms.

In a preferred embodiment the at least one at least bidentate organic compound is derived from a di-, tri- or tetracarboxylic acid or a monocyclic, bicyclic or polycyclic ring system which is derived from at least one heterocycle selected from the group consisting of pyrrole, alpha-pyridone and gamma-pyridone.

In a particularly preferred embodiment the at least one at least bidentate organic compound is a di- or tricarboxylic acid or substituted or unsubstituted imidazole.

Particular preference is given to using imidazoles such as 2-methylimidazole, acetylenedicarboxylic acid (ADC), camphordicarboxylic acid, fumaric acid, succinic acid, benzenedicarboxylic acids such as phthalic acid, isophthalic acid, terephthalic acid (BDC), aminoterephthalic acid, triethylenediamine (TEDA), naphthalenedicarboxylic acids (NDC), biphenyldicarboxylic acids such as 4,4'-biphenyldicarboxylic acid (BPDC), pyrazinedicarboxylic acids such as 2,5-pyrazinedicarboxylic acid, bipyridinedicarboxylic acids such as 2,2'-bipyridinedicarboxylic acids such as 2,2'-bipyridine-5,5'-dicarboxylic acid, benzenetricarboxylic acids such as 1,2,3-, 1,2,4-benzenetricarboxylic acid or 1,3,5-benzenetricarboxylic acid (BTC), benzenetetracarboxylic acid, adamantanetetracarboxylic acid (ATC), adamantanedibenzoate (ADB), benzenetribenzoate (BTB), methanetetrabenzoate (MTB), adamantanetetrabenzoate or dihydroxyterephthalic acids such as 2,5-dihydroxyterephthalic acid (DHBDC) as at least bidentate organic compounds.

Very particular preference is given to, inter alia, 2-methylimidazole, 2-ethylimidazole, phthalic acid, isophthalic acid, terephthalic acid, 2,6-naphthalenedicarboxylic acid, 1,4-naphthalenedicarboxylic acid, 1,5-naphthalenedicarboxylic acid, 1,2,3-benzenetricarboxylic acid, 1,2,4-benzenetricarboxylic acid, 1,3,5-benzenetricarboxylic acid, 1,2,4,5-benzenetetracarboxylic acid, aminoBDC, TEDA, fumaric acid, biphenyldicarboxylate, 1,5- and 2,6-naphthalenedicarboxylic acid, tert-butylisophthalic acid, dihydroxybenzoic acid.

In particular, preference is given to 2-methylimidazole, terephthalic acid, 2,6- and 1,5-naphthalenedicarboxylic acid, isophthalic acid, fumaric acid, 1,3,5-benzenetricarboxylic acid, trimellitic acid, glutaric acid, 2,5-dihydroxyterephthalic acid and 4,5-imidazoledicarboxylic acid and also acids derived therefrom. Also preferred is formate as at least bidentate organic compound.

Very particular preference is given to fumaric acid, terephthalic acid, 1,3,5-benzenetricarboxylic acid and 2-methylimidazole.

In addition to these at least bidentate organic compounds, the metal organic framework can further comprise one or more monodentate ligands and/or one or more at least bidentate ligands which are not derived from a dicarboxylic, tricarboxylic or tetracarboxylic acid or from a monocyclic, bicyclic or polycyclic ring system which is derived from at least one heterocycle selected from the group consisting of pyrrole, alpha-pyridone and gamma-pyridone.

The pore size of the metal organic framework can be controlled by selection of the appropriate optional monodentate ligand and/or the at least bidentate organic compound. It is frequently the case that the larger the organic compound, the larger the pore size. The pore size is preferably from 0.2 nm to 30 nm, particularly preferably in the range from 0.3 nm to 3 nm, based on the crystalline material.

Examples of metal organic frameworks can be found in WO 2009/09277 in the Table (page 11 to 23), wherein - in addition to the designation of the MOF - the metal and the at least bidentate ligand, the solvent and the cell parameters

(angles alpha, beta, and gamma and the dimensions A, B and C in Å) are indicated. The latter were determined by X-ray diffraction.

Further metal organic frameworks are MOF-2 to 4, MOF-9, MOF-31 to 36, MOF-39, MOF-69 to 80, MOF103 to 106, MOF-122, MOF-125, MOF-150, MOF-177, MOF-178, MOF-235, MOF-236, MOF-500, MOF-501, MOF-502, MOF-505, IRMOF-1, IRMOF-61, IRMOP-13, IRMOP-51, MIL-17, MIL-45, MIL-47, MIL-53, MIL-59, MIL-60, MIL-61, MIL-63, MIL-68, MIL-79, MIL-80, MIL-83, MIL-85, CPL-1 to 2, SZL-1 which are described in the literature.

Preferred metal organic frameworks are MIL-53, Zn-tBu-isophthalic acid, Al-terephthalate, MOF-5, IRMOF-8, Cu-1,3,5-benzenentricarboxylate, Al-2,6-naphthalenedicarboxylate, Al-amino-terephthalate, , Al-1,3,5-benzenentricarboxylate, Al-2,6-naphthalenedicarboxylate, Mg-2,6-naphthalenedicarboxylate, Al-fumarate, Zn-2-methylimidazolate, Zn-2-aminoimidazolate, , MOF-177, MOF-74, MOF-205, UiO66, MOF801, MOF808, Zn-dihydroxyterephthalate, Mn-terephthalate, Mg-formate, Fe-1 ,3,5-benzenetricarboxylate

More preferred metal organic frameworks are Al-terephthalate, Al-fumarate, Al-1,3,5-benzenetricarboxylate, , Mg-NDC, Mg-formate, MOF-74, MOF-5, MOF-177, MOF-205, IRMOF-8, Cu-1,3,5-benzenetricarboxylate and Zn-2-methylimidazolate,

More preferred are Al-terephthalate, MOF-177, MOF-205, IRMOF-8, Cu-1,3,5-benzenetricarboxylate, Al-fumarate and Zn-2-methylimidazolate.

More preferred are Cu-1,3,5-benzenetricarboxylate, also referred to as Basolite™ C300 or HKUST-1, Zn-2-methylimidazolate, also referred to as ZIF-8 or Basolite ™ Z1200, Al-fumarate, also referred to as Basolite™ A520, Al-terephthalate, also referred to as MIL-53 or Basolite™ A100, and Mg-formate, also referred to M050; more preferred are Cu-1,3,5-benzenetricarboxylate, Al-fumarate and Zn-2-methylimidazolate and Mg-formate (M050); and particularly preferred is Al-fumarate.

Process for Conditioning

The present invention relates to a process for conditioning a raw suspension SR comprising at least one metal-organic framework as described above and at least one suspension medium SM1, wherein the raw suspension SR is conditioned by means of at least one membrane filtration, to obtain a product suspension SP, comprising the at least one metal-organic framework and at least one suspension medium SM2.

Raw Suspension SR

The term "suspension" for the purpose of the invention relates to a heterogeneous mixture of solid particles comprising at least one meal-organic framework and at least one suspension medium, wherein the at least one suspension medium is at least one liquid.

The term "raw suspension SR" refers to a suspension, which is directly obtained from method for the preparation of at least one metal-organic framework.

Preferably, the raw suspension SR according to the invention is obtained by a method for the preparation of the at least one metal-organic framework, comprising

- reacting at least one metal salt with at least one at least bidentate organic compound, preferably in the presence of at least one additional compound AC, in a reaction medium, wherein the reaction medium corresponds to the at least one suspension medium SM1.

The person skilled in the art is able to select the appropriate reaction conditions such as reaction temperature, reaction medium, suitable additional components such as at least one base or at least one acid, template components, and a suitable reaction pressure.

In a preferred embodiment, the preparation of the at least one metal-organic framework is conducted in the presence of at least one additional compound AC.

The at least one additional compound AC according to the invention is at least one template compound, at least one base or at least one acid, preferably at least one base.

The reaction medium according to the above-described method for the preparation of at least one MOF comprises typically at least one liquid and corresponds to the suspension medium SM1 present in the raw suspension SR.

The term "product suspension SP" refers to a suspension, which is obtained after the present process for conditioning the raw suspension SR. The end of said process is defined as the moment, when the desired concentration and /or purity of the at least one metal-organic framework in the suspension medium SM2 is obtained.

The term "suspension medium" refers to the liquid phase in which the at least one metal-organic framework is present. Suspension medium SM1 refers to the liquid phase comprised in the raw suspension SR, while suspension medium SM2 refers to the liquid phase comprised in the product suspension SP.

In a preferred embodiment of the present process, the at least one metal-organic framework is insoluble in the particular suspension medium. Preferably, less than 10 g metal-organic framework are soluble in 1 liter of suspension medium, preferably less than 1 g/l and particularly preferably less than 0.1 g/L at 20°C. In the case the suspension medium comprises more than one metal-organic framework, the total amount of metal-organic frameworks soluble in 1 L of the suspension medium is preferably less than 10 g, preferably less than 1 g and particularly preferably less than 0.1 g.

The suspension medium comprises one or more liquids, for example 2, 3, 4 or 5 liquids. In the case the suspension medium comprises more than one liquid, it is preferred that the particular liquids are miscible with each other. Examples for such a mixture of more than one liquid is a mixture of water and one or more alcohols, selected from the group consisting of methanol, ethanol, i-propanol, n-propanol, and n-butanol, iso-butanol and sec-butanol, or a mixture of two or more alcohols selected from the above-defined alcohol, for example a mixture of methanol and ethanol.

According to the invention suspension medium SM1 and suspension medium SM2 are independently from each other selected from water, methanol, ethanol, i-propanol, i-butanol and sec-butanol a mixture of two or more thereof.

In one embodiment of the invention suspension medium SM1 differs from suspension medium SM2.

In a preferred embodiment, suspension medium SM1 and suspension medium SM2 are equal, in a particularly preferred embodiment suspension medium SM1 and suspension medium SM2 are water.

Preferably, suspension media SM1 and SM2 comprise independently from each other at least 80 % by weight, more preferably at least 90 % by weight, more preferred at least 95 % by weight, particularly preferred at least 98 % by weight of water, based on the total amount of the particular suspension medium. In a particularly preferred embodiment of the invention the suspension media SM1 and SM2 consists of water.

The raw suspension SR may comprise several further components. Typical further components are for example unreacted starting materials; i.e. unreacted metal salt, organic compound or optionally additional compound AC; impurities contained in said starting materials and side-products, such as salts.

In one preferred embodiment of the invention, the raw suspension SR is free from such further components. This applies especially in cases, wherein no salt is produced as a by-product of the reaction of at least one metal salt with at least one at least bidentate organic compound. One example is - in general - the reaction of a metal hydroxide with a dicarboxylic acid, wherein the MOF is formed under dehydration.

In another preferred embodiment of the invention, the raw suspension SR comprises at least one salt, which is formed as a side-product of MOF-preparation. A typical method refers to the reaction of at least one at least bidentate organic compound, for example fumaric acid, with at least one metal salt, for example aluminum sulfate, under addition of at least one base, for example sodium hydroxide, in at least one reaction medium corresponding to suspension medium SM1, for example water. The resulting raw suspension SR comprises at least Al-fumarate and sodium sulfate.

In a particularly preferred embodiment, the raw suspension SR comprises at least one salt. It is preferred that said salt is soluble in the suspension medium SM1. Soluble in this context means, that at least 33 g, preferably at least 50 g and particularly preferably at least 100 g of the salt are soluble in 1 L of the suspension medium SM1 at 20°C.

The term "conditioning" for the purpose of the invention relates to methods for the purification and/or concentration of the raw suspension SR according to the invention and comprises at least one membrane filtration.

"Membrane filtration" for the purpose of the present invention relates to microfiltration or ultrafiltration, preferably microfiltration. The general principle of membrane filtrations is well-known for the person skilled in the art. However, in the following paragraphs further definitions are given to describe the present method in more detail.

The term "purification" for the purpose of the present invention refers to removing further components optionally present in the raw suspension SR, preferably removing at least one salt formed in the above described method. The present process for conditioning can comprise one or more purification steps, wherein at least one purification step is carried out as membrane filtration. Preferably, all purification steps are carried out as membrane filtration.

Accordingly, the term "purification step" refers to a membrane filtration after which the MOF-containing suspension comprises a decreased amount of optionally further components, preferably of at least one salt, than before the membrane filtration.

In a typical embodiment of the invention, the raw suspension SR comprises from 1 to 25 % by weight, preferably 2 to 20 % by weight and particularly preferably 5 to 10% by weight of at least one salt, based on the total weight of the raw suspension SR. In a preferred embodiment the product suspension SP comprises less than 2 % by weight, preferably less than 1.0 % by weight, more preferably less than 0.3 % by weight, and particularly preferably less than 0.2 % by weight of the at least one salt, wherein all given amounts are based on the total weight of the product suspension SP.

The term "concentrating" according to the invention refers to increasing the concentration of at least one metal-organic framework in the MOF-containing suspension. The present process fro conditioning comprises one or more concentration steps, wherein at least on concentration step is carried out as membrane filtration. Preferably, all concentration steps are carried out as membrane filtration. Accordingly, the term "concentration step" preferably refers to a membrane filtration after which the MOF-containing suspension comprises a higher concentration of the at least one metal-organic framework than before the particular membrane filtration.

Typically, the raw suspension SR according to the invention comprises 1 to 25 % by weight, preferably 5 to 20

% by weight, particularly preferably 5 to 10 % by weight of at least one MOF, based on the total weight of the raw suspension SR.

In a preferred embodiment the at least one membrane filtration comprises at least one purification step, or at least one concentration step, or at least one purification step and at least one concentration step.

In the case, the raw suspension SR does not comprise any salts as a side-product of the MOF-preparation or the side-products have no disadvantageous effect on the further application of the MOF, the conditioning can be carried out without purification steps 8e.g. the preparation of Basolite M050 - magnesium formate).

Accordingly, in one embodiment of the invention the at least one membrane filtration comprises only at least one concentration step and no purification step.

In the case, the method for the preparation of the at least one MOF results in a raw suspension SR, wherein the final concentration of MOF is already in a suitable range for a further application, the conditioning can be carried out without concentration steps.

Accordingly, in one embodiment of the invention the at least one membrane filtration comprises only at least one purification step and no concentration step.

In a preferred embodiment the at least one membrane filtration comprises at least one purification step and an at least one concentration step.

As described above the term "membrane filtration" for the purpose of the present invention refers to microfiltration or an ultrafiltration. Membrane filtration preferably refers to microfiltration, preferably as crossflow-filtration.

Cross-flow filtration is where the fluid is passed through tangentially with respect to the membrane. Part of the feed stream containing the treated liquid is collected below the filter while parts of the suspension medium are passed through the membrane untreated. Cross flow filtration is understood to be a unit operation rather than a process. The microfiltration process is pressure driven with suspended particles, i.e. MOF-particles and a suspension medium as retentate and dissolved solutes, for example at least one salt plus suspension medium as permeate. The use of hydraulic pressure accelerates the separation process by increasing the flow rate (flux) of the liquid stream but does not affect the chemical composition of the species in the retentate and product streams.

In a preferred embodiment at least one membrane filtration is carried out as a diafiltration. Accordingly, it is preferred that the at least one purification step and/or the at least one concentration step is carried out as diafiltration.

For the purpose of the present invention the term "diafiltration" refers to a membrane filtration in which a diafiltration medium is added to the retentate before or during the membrane filtration. The addition of the diafiltration medium generally does not alter the concentration of the metal-organic framework. The diafiltration medium comprises a liquid phase which is equal or different from the suspension medium SM1.

In the case the diafiltration medium differs from the suspension medium SM1, the suspension medium SM1 of the raw suspension SR can be at least partly, preferably completely replaced by a different type of suspension medium, i. e. by the diafiltration medium. Accordingly, suspension medium SM2 corresponds to a mixture of suspension medium SM1 and infiltration medium, in the case SM1 is partly replaced by the infiltration medium, and suspension medium SM2 corresponds to the diafiltration medium, in the preferred case, wherein suspension medium SM1 is completely replaced by the diafiltration medium.

The diafiltration medium is preferably miscible without a phase boundary with the suspension medium SM 1. The diafiltration medium is particularly preferably miscible without a phase boundary with the previous suspension medium in any ratio. Here, miscible without a phase boundary means that no phase boundary, for example in the form of an emulsion, is formed under the prevailing pressure and temperature conditions in each case and at the mixing ratios used.

In a preferred embodiment the diafiltration medium is selected from the group consisting of water, methanol, ethanol, n-propanol, iso-propanol, n-butanol, iso-butanol or sec-butanol or a mixture of two or more thereof, particularly preferred is water.

In a particularly preferred embodiment of the invention the diafiltration medium is equal to the suspension medium SM1 in the raw suspension SR. In said embodiment, suspension media SM1 and SM2 are equal. Particularly preferably, the suspension media SM1 and SM2 and the diafiltration medium are water.

As described above, the membrane filtration comprises preferably at least one concentration step, wherein the term "concentration step"refers to a membrane filtration after which the metal-organic framework is present in a smaller volume of suspension medium than before the filtration. For example, the suspended metal-organic framework can be concentrated by removing part of the suspension medium SM1 via a membrane, with the suspended metal-organic framework remaining in a smaller volume of the suspension medium. Alternatively, the concentration can be altered by adding more raw suspension SR in the feed. Alternatively, the concentration can be altered by membrane filtration carried out without feed flow.

The term "retentate" refers to the stream which does not go through the membrane, while the term "permeate" refers to the stream which goes through the membrane. The resulting mass flow per unit area of the membrane is referred to as permeate flux and is generally reported in $kg/m^2/h$. In accordance with the definition of retentate and permeate,

the retentate side is the side of the membrane on which the raw suspension SR is present and the permeate side is, correspondingly, the side of the membrane on which the liquid phase is present which is essentially free from the at least one metal-organic framework.

The progress or degree of replacement of the suspension medium SM1 is indicated by the replacement coefficient. The replacement coefficient is the amount of permeate taken off or the amount of diafiltration medium introduced (amount of permeate separated off and amount of diafiltration medium introduced are identical in the case of diafiltration) divided by the amount of retentate. The amounts can here be determined either consistently by volume or consistently by weight. The fresh diafiltration medium can be introduced in liquid or solid form. In the case of solid form, it can be present, for example, in a crystalline state or a frozen state and can liquefy or be liquefied while carrying out the method.

After carrying out the method of the invention, i.e. after the last membrane filtration, the at least one metal-organic framework is present in suspension medium SM2 as defined above, preferably in water.

The process of the invention preferably comprises at least one concentration step, particularly preferably two. The first concentration step is generally carried out before the first diafiltration step. Here, the metal-organic framework is concentrated in the reaction medium. As a result, less diafiltration medium is consumed or permeate is produced in the subsequent diafiltration steps for the required depletion than is the case in direct diafiltration of the unconcentrated raw suspension. The second concentration step is generally carried out after the last diafiltration step. Here, final concentration is effected, if appropriate, with the aid of one or more suitable auxiliaries.

During the process, diafiltration and concentration steps can be arranged in series in any suitable number and order.

The optimal number of diafiltration and concentration steps can be determined by a person skilled in the art for the respective MOF as a function of the type and amount of impurities.

In general, the retentate formed in each filtration is passed onto the next filtration. The essentially MOF-free permeate obtained from each individual filtration can be worked up separately or as collected permeate to recover, for example salt, suspension medium, diafiltration medium and/or auxiliaries. The materials recovered from the individual permeates or the collected permeate can either be fed back into the membrane filtration unit at a suitable point, e. g. to increase the concentration of MOF in the suspension medium or can be utilized in another way. The permeate respectively the collected permeate can be directly used in several application, preferably in methods for coating a substrate as described in more detail below.

In a preferred embodiment, the product suspension SP comprises from 10 to 30 % by weight, preferably from 15 to 25% by weigh and in particular from 18 to 22% by weight of at least one metal-organic framework, based on the total amount of the product suspension SP.

The membrane filtration process is advantageously carried out as a microfiltration (crossflow filtration) or an ultrafiltration, depending on the particle size of the MOF to be conditioned.

The raw suspension is filtered in a membrane filtration unit which can be connected in a fixed manner for at least part of the time to a reactor. The membrane filtration unit can be made up of only one filtration unit element or a plurality of filtration unit elements but comprises at least one membrane filter. It generally runs from one or more reactors to the offtake point of the conditioned MOF suspension.

The chosen number and type of filtration unit elements can be matched by a person skilled in the art to the respective needs. In this way, it is possible to obtain membrane filtration units having a complex structure. Preference is given to simple and therefore cheap membrane filtration units. For the purposes of the present invention, filtration unit elements are each a detachable part of the membrane filtration unit which performs a function in the membrane filtration unit. The individual filtration unit elements comprise, for example, pipe and/or hose connections, seals, pressure and temperature meters and regulators, pumps, valves, filters and also feed lines and discharge lines. An appropriate membrane filtration unit can comprise one, more than one or all of the elements mentioned by way of example, with one or more of each being present in a suitable embodiment and order. The membrane filtration unit can also comprise parallel lines, filters and filtration paths which can be operated simultaneously or alternately.

The membrane filters used comprise a filter housing which is stable under the respective conditions of the filtration. Such conditions are, for example, pressure, temperature or the type and composition of catalyst suspension. A filter housing can comprise one or more membranes. A filter housing is a vessel comprising one or more membranes in a suitable orientation and can be connected in a fixed manner for at least part of the time to one or more other filtration unit elements. The membrane can be fixed permanently in the filter housing or can be removable. The membrane can have a flat, disk-like, tubular, capillary or rolled geometry. Depending on the area needed, a filter can comprise a plurality of membranes arranged in parallel or in series. In a preferred embodiment, use is made of a ceramic membrane which is preferably inserted with the aid of elastomer seals in metal filter housing or a metal membrane which is preferably fixed in the filter housing. The ceramic membrane preferably has a tubular or multichannel geometry and the metal membrane preferably has a tubular geometry.

A membrane generally comprises only one separation layer (homogeneous self-supporting membrane) or a separation layer and at least one support structure (multilayer composite membrane). The use of a thin separation layer

which is not mechanically stable on a single-layer or multilayer porous support structure generally increases the permeate flux and ensures mechanical strength of the membrane. This support structure can consist of the same material as the separation layer or of at least one different material. It generally has coarser pores than the separation layer.

Possible material combinations of separation layer and support structure can be, for example: metal-metal, ceramic-metal, ceramic-ceramic, ceramic-carbon, polymer-polymer, polymer-metal, polymer-ceramic, polymer-ceramic on metal, carbon-carbon, carbon-metal, carbon-ceramic. Homogeneous, self-supporting membranes based on polymers, carbon, ceramic or metal can also be used.

As organic separation layer and/or support structure materials, it is possible to use, for example, polytetrafluoroethylenes, polyvinylidene fluorides, polyethylene, polypropylene, polysulfones, polyether sulfones, polyether ketones, polyamides, polyesters, polyacrylonitriles, regenerated celluloses, silicones, polyimides or materials having an equivalent function.

Examples of inorganic separation layer and/or support structure materials are: alpha-$Al_2O_3$, gamma-$Al_2O_3$, $ZrO_2$, $TiO_2$, SiC, mixed ceramic materials, stainless steel, nickel, metal alloys and carbon. Examples are (MF=microfiltration membrane, UF=ultrafiltration membrane):

| Manufacturer | Membrane | Separation limit (kD) Pore diameter (nm or μm) |
|---|---|---|
| Atech innovations | UF/$TiO_2$ on alpha-$Al_2O_3$/1, 2 | 20 kD |
| GmbH | UF/$TiO_2$ or Z$ZrO_2$ on alpha- $Al_2O_3$/1, 2 | 50 nm |
| | MF/alpha-$Al_2O_3$ on alpha- $Al_2O_3$/1, 2 | 0.1; 0.2; 0.4;0.8; 1.2 μm |
| Rhodia/Orelis | MF/$ZrO_2$ or $TiO_2$ on ceramic/1, 2 | 0.1; 0.2; 0.45; 0.8 μm |
| | UF/$ZrO_2$ or $TiO_2$ on ceramic/1, 2 | 15, 50; 150; 300 kD |
| | UF/$ZrO_2$-$TiO_2$ on carbon/1 | 50; 150; 300 kD |
| | MF/$ZrO_2$-$TiO_2$ on carbon/1 | 0.14 μm |
| Graver technologies | UF/$TiO_2$ on steel/1 | 100 nm |
| GKN Sinter Metals | MF/metal on metal/1, 3 | 0.3-1 μm |
| Microdyn NADIR | UF/polyether sulfone or polysulfone/3 | 10-150 kD |
| GmbH | UF/polyether sulfone/1 | 49, 100 kD |
| | MF/polyethylene or polypropylene, self-supporting without support structure/1 | 0.2 and 1 μm |
| Creavis | UF/$ZrO_2$ on alpha-$Al_2O_3$ and metal/3 | 25, 80 nm |
| GE-Osmonics | UF/polysulfone/3 | 40 nm |
| | UF/PVDF/3 | 10 kD |
| | MF/PVDF/3 | 300 nm |
| Pall | UF/$TiO_2$ or $ZrO_2$ on ceramic/1, 2 | 5, 10, 50 nm |
| Schuhmacher | MF/alpha-$Al_2O_3$ on ceramic 1, 2 | 100 and 200 nm |
| Inocermic | UF/$ZrO_2$ on ceramic(1, 2 | 3nm |
| Saint-Gobain | MF/SiC on SiC/1, 2 (ASiC = recrystallized SiC) | 250, 500, 800, 1500, 3000 nm |

1: tubular membrane;
2: multichannel element
3: flat membrane for rolled, pocket, stacked plate or special modules having a moved membrane or stirring devices between the membranes

The pore diameter of the separation layer generally depends mainly on the respective size of the smallest MOF-particles. When a suitable pore size of the separation layer is selected, MOF-losses due to turbid outflow can be disregarded. For the purposes of the present invention, the term turbid outflow refers to MOF-particles which can pass through the membrane in the case of a badly chosen pore size of the separation layer and therefore appear in the permeate. This turbid outflow and associated blocking of the filtration medium occurs to an increased extent when using classical filtration processes for removal of solids, e.g. using filter presses, pressure filters, candle filters, centrifuges and disk filters, and also for concentrating solids, e.g. using separators, centrifuges and decanters.

The pore size of the separation layer should advantageously be smaller than the size of the smallest MOF particles. If the MOF particles are present in aggregated form, the preferred pore size depends not on the size of the smallest catalyst particles but on the size of the smallest aggregates. The reported pore size of the separation layer is

generally based on the diameter for which 90% of the pores are smaller than the pore size indicated. The separation layer of the membrane is the layer of the membrane comprising the smallest pores of the membrane. The pore size or the separation limit of the membranes is determined by methods known to those skilled in the art.

Depending on the size distribution of the MOF particles, the separation layers of the membrane used preferably have a pore diameter in the range from 50 nm to 800 nm, in more preferably in the range from 50 nm to 500 nm and particularly preferably in the range from 50 nm to 200 nm

When choosing the membrane, it is necessary to take account not only of the pore size of the separation layer and the size distribution of the MOF particles, but also of the filtration conditions such as the composition of the suspension medium or the diafiltration medium, the pressure conditions, the temperature and any auxiliaries present. A suitable membrane advantageously does not change its separation properties, i.e. its separation limit, significantly under any of the factors mentioned by way of example.

The separation limit in kD is the molecular weight of a dissolved test substance which has a retention (R) of 90%. Here, R is 1 minus the concentration in the permeate, divided by the concentration in the retentate.

The correlation between separation limit in kD and the pore diameter is known to those skilled in the art. Thus, for example, pore sizes of from 2.0 to 1.2 $\mu$m are used for molecular weights greater than 1000 kD, pore sizes of 0.6 $\mu$m are used for 1000 kD, 0.2 $\mu$m is used for 400 kD, 0.1 $\mu$m is used for 200 kD, 50 nm is used for 100 kD and 10 nm is used for 20 kD.

In the method of the invention, the conditions of the filtration, e.g. pressure, temperature or flow velocity, can be selected independently of the conditions in the reaction system if required. This independence allows the conditions to be optimized for the respective filtration. For example, the temperature and/or the pressure can be increased or decreased, the flow velocity can be selected optimally, the suspension medium can be replaced and/or the properties and composition of the retentate and/or permeate can be optimized by addition of auxiliaries. A person skilled in the art can choose the filtration conditions which are optimal in each case. Further information on this subject may be found in the technical literature.

The filtration can be carried out at various pressures and can be individually selected and optimized for each filtration of the method. Thus, they can be identical to or different from the pressures during a preceding or subsequent filtration and can change or be altered for each filtration of the method even during the filtration.

Various pressures, which are given different names, generally prevail in a filter during a filtration. The permeate pressure is the pressure on the permeate side of the membrane and the retentate pressure is the pressure on the retentate side of the membrane. The filtration can be carried out at a retentate pressure in the range from 1 to 100 bar, preferably in the range from 1 to 50 bar. The transmembrane pressure is the retentate-side arithmetic mean of the pressures at the inlet to the filter and at the outlet from the filter, reduced by the permeate pressure. It can likewise be optimized for each filtration and be identical to or different from the transmembrane pressure in other filtrations of the method. The transmembrane pressure can be, for example, in the range from 0.1 bar upward, in particular in the range from 0.2 to 50 bar, preferably in the range from 0.5 to 25 bar, and particularly preferably in the range from 1 to 20 bar. The pressure conditions to be selected are essentially dependent on the type of membrane, the diameter of the pores of the membrane, the catalyst concentration, the hydrodynamic conditions in the membrane module and the mechanical stability of all filtration unit elements. Higher transmembrane pressures generally lead to higher permeate fluxes. The pressure required can be generated by pumping and/or by gravity.

The temperature can be selected and optimized independently for each filtration carried out during the method of the invention. Higher temperatures generally lead to higher permeate fluxes. It can consequently be identical to or different from the temperature in the preceding and/or subsequent filtration step. The value chosen depends mainly on the solubility and the melting and boiling points of the respective materials present under the pressure conditions prevailing in each case. It is possible to choose temperatures in the range from 0 to 200 °C, advantageously in the range from 10 to 180 °C, preferably in the range from 20 to 150 °C, and particularly preferably in the range from 20 to 80 °C.

In a filtration, the conditions are preferably set so that only a thin or not noticeable covering layer is formed on the membrane. Covering layers comprise for example a solid material which deposits on and/or in the membrane and can adversely affect the filtration. This material can be any solid material which is present in the raw suspension. For example, it can comprise the metal-organic framework. In the case of filters and membranes having a flat or disk-like membrane geometry, stirring and/or shearing elements and/or movements of the membrane or of the filter, for example by means of rotation or vibration modules, can also be used for preventing covering layers. Furthermore, a high flow velocity of the MOF suspension and associated shear at the membrane surface can be used. In general, a flow velocity in the range from 0.2 to 20 m/s is selected. The appropriate flow velocity depends on the filter used and the membrane used.

Filters and membranes having a rolled geometry are preferably used at a flow velocity in the range from 0.2 to 2 m/s. Filters and membranes having a tubular geometry are used at a preferred flow velocity in the range from 1 to 6 m/s. Rotary filters are preferably used at a flow velocity in the range from 5 to 20 m/s. If a plurality of possibly different filters is used, the flow velocity can be matched to the respective filter.

A person skilled in the art will have available suitable means of measuring and controlling the flow velocities.

To remove covering layers, the method of the invention also allows flow reversal, which can be brought about by increasing the permeate pressure above the retentate pressure (permeate backflushing).

The above-described measures for reducing or preventing covering layers can be used individually, in combination or alternately. Covering layers can be influenced in each filter of the membrane filtration unit by means of identical or different measures or identical or different combinations of measures.

A further preferred embodiment of the method of the invention comprises addition of one or more suitable auxiliaries. For the purposes of the invention, the term auxiliary refers to all solid, liquid or gaseous materials which are added to the raw suspension SR before or during filtration and are suitable for positively influencing the filtration. The filtration can, for example, be influenced favorably by lowering the viscosity. Likewise, an auxiliary or a plurality of auxiliaries can be used for stabilizing the raw suspension SR. Here, stabilization of the raw suspension SR can relate to physical parameters such as the settling velocity of the MOF particles and/or chemical parameters such as the pH. The use according to the invention of at least one auxiliary enables, for example, higher final concentrations of the MOF in the retentate to be achieved.

The method of the invention can be operated continuously or discontinuously. In discontinuous operation, the raw suspension SR is placed in a vessel which functions as circulation vessel in a pumped circuit into which one or more filters are integrated. The raw suspension SR can be circulated by pumping, permeate can be taken off and, if appropriate, liquid or solid materials such as auxiliaries or suspension media can be introduced at a suitable point in the pumped circuit. Points which are suitable for this purpose are, for example, lines or vessels upstream of filters or else the filters themselves. The raw suspension SR is circulated by pumping until the desired concentration of the metal-organic framework and/or the desired purity of the suspension medium has been achieved. Thus, for example, the MOF concentration can be altered by removing liquid constituents by filtration or by adding some or all constituents of the suspension medium in the same or different composition. Furthermore, qualitative changes can be produced by, for example, altering the chemical or physical properties of the MOF suspension, e.g. pH or viscosity, by addition of one or more auxiliaries. Furthermore, parameters such as temperature or pressure can be altered. These changes can in each case be carried out alone or in combination.

When the method is carried out continuously, the raw suspension SR taken from the reaction system is passed once through or along one or more filters of the membrane filtration unit. Here, all the measures mentioned by way of example above for altering the concentration of the MOF or the concentration or properties of the raw suspension SR can be used either alone or in combination.

In summary, the conditioned MOF can be taken off as product suspension SP, preferably an aqueous product suspension SP, particularly preferably as a product suspension SP consisting essentially of MOF and water and also, if applicable, added dispersant or surfactant from the membrane filtration unit after the last filtration and be used directly in any applications, for example in a method for coating a substrate with at least one metal-organic framework.

Compared to existing methods of conditioning a raw suspension comprising at least one MOF, the invention has the particular advantage that the conditioning process needs less energy and produces less waste water. The element of diafiltration makes it possible to take the MOF in high purity in any desired suspension medium in a desired concentration from the membrane filtration unit.

It is also advantageously that the MOF is provided in the form of a product suspension SP which can be directly used in further applications, especially in coating processes.

Accordingly the present invention provides a method for coating at least part of a surface of a substrate with an active layer, comprising at least one metal-organic framework, comprising

a) bringing at least part of the surface of the substrate into contact with a coating composition, wherein the coating composition comprises the product suspension as defined above, comprising the at least one metal-organic framework, and at least one binder.

In a preferred embodiment the method further comprises

b) drying the coated substrate obtained in step a).The preferred embodiments of the at least one metal-organic framework and the product suspension SP correspond to the preferred embodiments described above in view of the inventive process for conditioning.

The at least one binder can be a water-based or solvent-based material. For example, the at least one binder is selected from polyacrylamide, polyacrylate, polytetrafluoroethylene, polyvinylidene fluoride and a polyalkyleneimine (e.g., polyethylenimine). In a preferred embodiment, the binder component is a water-based material, styrene acrylic polymers, e. g. Joncryl 3030 (BASF SE).

The term "substrate" refers to one or more materials selected from a metal, polymer (including plastics), paper, glass, ceramics, woven fabric, non-woven fabric, fiber composite material and composite materials of any of the foregoing

(e.g., polymer coated metal). The substrate can be a rigid three-dimensional object (e.g., a component of a heat transfer device, a jar, bottle, can, container, drum, tote or intermediate bulk container, foam) made of a metal or plastic, a flexible foil (e.g., polypropylene, polypropylene fibers, polyethylene, polystyrene, polyethylene terephthalate, polyethylene terephthalate fibers, polyester, flash-spun high-density polyethylene, molded fiber, aluminum, stainless steel, aluminum coated polymer, nylon polyamide, pressed paperboard, or biodegradable versions thereof or a flexible fabric (e.g., woven and non-woven materials, cotton fabric, paper, tissues, polyethylene fabrics, polyethylene terephthalate fabrics, glass fiber, carbon fiber).

In a preferred embodiment the substrate is a heat transfer element. For example, the substrate can be a chiller component, a heat pump component, a heating, ventilating and air conditioning (HVAC) component, a packaging component, an odor removal component, a radiator component, a fin of a heat transfer device, or a refrigerator component.

The method for coating can be carried out with or without a primer layer. The method can include forming a primer layer and forming the active layer on the primer layer. Forming the active layer on the primer layer can include coating the primer layer with the active layer.

In another preferred embodiment the method comprises coating the substrate with a primer layer. Such a primer layer is coated onto the surface of the substrate before the metal-organic framework is coated onto the primer layer. Suitable primers are described e.g. in WO 2018/036997 A1.

The term "active layer" refers to the MOF-comprising layer on the substrate, in other words to the MOF-film coated onto the substrate.

Accordingly, in a preferred embodiment the inventive method comprises the steps of

a1) coating a primer layer onto at least part of the surface of the substrate,

a2) bringing at least part of the surface of the substrate obtained in step a1) into contact with the coating composition, wherein the coating composition comprises the product suspension SP as described above, comprising at least one metal-organic framework, and at least one binder; and

a3) preferably drying of the coated substrate obtained in step a2).

The primer layer can include any suitable material such as a polyalkylenimine. The polyalkylenimine can be branched, straight or a combination thereof, for example, the primer layer can include a branched polyalkylenimine. The polyalkylenimine can have a charge density of about 1 to about 35 meq/g and the molecular weight of the polyalkylenimine can be from about 20,000 Daltons to about 3,000,000 Daltons or from about 500,000 Daltons to about 1,000,000 Daltons. According to certain example aspects, the alkylene moiety of the polyalkylenimine can be ethylenimine, 1,2-propylenimine, 1-2-butylenimine, 2,3-butylenimine or a combination thereof. For example, the polyalkylenimine can be a polyethylenimine, such as, a branched polyethyleneimine. In certain aspects, the branched polyethylenimine can be highly branched.

The primer layer (with or without the binder) is preferably as thin as possible. For example, the primer layer can have a thickness of about 10 μm or less. In certain aspects, the primer layer can have a thickness of about 0.05 μm to about 5 μm.

The method can include forming the primer layer and forming the active layer on the primer layer. Forming the active layer on the primer layer can include coating the primer layer with the active layer. Forming the active layer on the surface of the substrate or on the the primer layer can include dip coating, spray coating, electrostatic spray coating, knife coating, curtain coating or slot-die coating, roll coating or spin coating. For example, a component of a heat pump can coated with the primer layer and then dip coated with the active layer.

Drying for the purpose of the invention refers to drying at a temperature in the range from 20 to 200°C, preferably from 50 to 150°C, and particularly preferably from 60 to 120°C.

According to the invention the MOF material is provided in the form of a product suspension, which is directly obtained by the above-described process for conditioning a raw MOF-suspension. In other words, in one preferred embodiment the coating composition corresponds to the products suspension.

In another preferred embodiment the coating composition corresponds to a mixture of the product suspension and at least one binder. Such coating composition typically is prepared by adding at least one binder to the product suspension. In a preferred embodiment, the coating composition comprises

- 50 to 99 % by weight of the product suspension; and
- 1 to 50 % by weight of at least one binder,

with the proviso that the coating composition comprises at least 10 % by weight of at least one MOF, wherein the given amounts are based on the total weight of the coating composition.

The resulting MOF-water-binder combination can be coated onto the primer layer or directly on at least part of the surface of the substrate using one or more of the aforementioned techniques.

The MOF-containing active layer can have a thickness of about 50 μm to about 500 μm, or about 100 μm to about 250 μm, or about 150 μm to about 200 μm, or about 100 μm to about 200 μm, or about 150 μm to about 250 μm. The combined thickness of the composite material including both the primer layer and the active layer can be about 100 μm to about 260 μm, or about 100 μm to about 250 μm, or about 100 μm to about 210 μm, or about 100 μm to about 200 μm, or about 100 μm to about 160 μm or about 100 μm to about 150 μm. The layer thickness can be analyzed by means of optical or SEM microscopy performed on the cross section of the coated specimen.

It has been found, that the inventive coating composition a higher viscosity and higher process and storage stabilities, than conventionally prepared coating compositions.

Moreover, it has been found, that the resulting active layer, in other words the MOF-film coated onto the surface has a surprisingly higher density than a MOF-film coated by means of a conventional coating composition, formulated from MOF-powder.

The present invention is illustrated by means of the examples and figures below.

## Examples

Figure 1: Schematic of the membrane unit

**Fig. 1** shows a sketch of a filter unit. The filter unit comprises a pumped circuit comprising a feed vessel, a pump 1, a thermostat and a membrane module. A pressure meter (PI) and a flow meter (FI) are integrated into the circuit upstream of the membrane filter. (LI) represents a level indicator for the feed vessel. In addition to the pumped circuit, the filter comprises a reservoir for the diafiltration medium or permeate, which can be metered into the feed vessel by means of pump 2. Permeate obtained can be discharged into a permeate container and weighed by means of a scale.

### A. Preparation of MOF

Example of Al Fumarate synthesis (1 h, 60°C)

Raw materials:

| | | | |
|---|---|---|---|
| Fumaric acid | 0.211 mol | 24.47 g | 24.47 g |
| Sodium hydroxide | 0.633 mol | 25.32 g | 25.32 g |
| Aluminum sulfate * 18 mol water | 0.105 mol | 70.0 g | 70.0 g |
| Water | 36.8 mol | 661.7 g | 661.77 g |

Aluminum sulfate was dissolved in 300 g of water in a beaker, and heated up to 60 °C. To this solution a mixture of 411.5 g of solution (at 60 °C) of fumaric acid, sodium hydroxide and water was added during the 58 minutes. During the pumping time a white precipitate appeared. Once the solutions was exhausted, white precipitate was filtered off and washed 3 times with 500 ml of demineralized water. Obtained filter cake was dried in the air at 100 °C until the material appears as dry powder, and transferred to a vacuum dried at 130 °C for the 16 h to remove residual water.

Preparation yields approximately 33.08 g of material.

### B. Microfiltration experiments

#### 1. General experimental setups

The membrane filtration experiments followed the basic schematic of Figure 1 and were conducted in batch operation mode. The slurry comprising the metal-organic framework was filled into a feed vessel and circulated continuously over a membrane module, from which a permeate stream was removed. To concentrate / purify ("diafiltrate") the solution, either additional MOF-containing slurry or VE water was added into the feed vessel. Further concentration of the MOF was enabled by having no feed flow, i.e. by reducing the total volume of the slurry in the filtration loop. During the experiment, all pressures (pressures before and after the membrane module), temperature and cross-flow velocity were controlled. Moreover, the conductivity in the feed vessel was measured; giving an indication about the progress of the purification.

Characteristic values for a membrane separation experiment are the total mass concentration factor (CF) and the diafiltration factor (DF).

CF relates the total permeate mass to the initial mass in the feed vessel.

$$CF = (m_{permeate} + m_{feed,initial}) / m_{feed,initial}.$$

By that definition, assuming a 100% retention of the MOF, the Concentration Factor CF represents the relative concentration of the Basolite A520 compared to its initial concentration (e.g. CF = 1 in the beginning, CF = 2 when $m_{permeate} = m_{feed,initial}$ and the solid concentration is doubled).

DF represents the "washing factor", i.e. relating the amount of added VE water to the batch size. By this definition, DF is equal to the total permeate mass divided by the initial feed.

$$DF = m_{permeate} / m_{feed,initial}$$

Further characteristics of the units are summarized in Table 1 below. It is important to note, that in membrane unit 2, a membrane pump that enables a gentler conveyance of the medium was applied, as compared to a centrifugal pump in membrane unit 1.

Table 1: Characteristics of the two membrane units applied for the lab-scale membrane experiments

| Criterion | Membrane unit 1 | Membrane unit 2 |
|---|---|---|
| Type of pump | Impeller-type centrifugal pump | Membrane pump |
| Max. pumping flow (L/h) | 600 | 600 |
| Applied for experiments | Lab-scale modules (single-channel ceramic) | Lab-scale and technical-scale modules (multi-channel ceramic) |
| Automation | Manual operation | Fully automated |

2. Experimental plan and process evaluation

In total, two batches with Basolite A520 slurry were applied. In Table 2 below, the analytical properties of these two batches are summarized. As can be seen, both batches showed Basolite A520 concentrations of approximately 4.5 wt.-% and $Na_2SO_4$ concentrations of approximately 5 - 6 wt.-%, based on the total weight of the batch. The concentrations were measured after insertion into the respective membrane units which lead to a slight dilution of the slurry.

Table 2: Properties of the initial Basolite A520 batches

| Parameter | Batch 1 | Batch 2 |
|---|---|---|
| Total solid concentration (wt.-%) | 10.0 - 10.1 | 10.0 |
| $Na_2SO_4$ concentration (wt.-%) | 5.2 - 5.6 | 5.2 |
| Basolite A520 concentration (wt.-%) | 4.5 - 4.8 | 4.8 |

The parameters of the conducted experiments are summarized in Table 3. In total, three different experiments were conducted, MF1", MF2" and MF3"; aiming at a variation of the type of membrane and aiming at a maximum concentration of the Basolite A520 slurry (using a maximum attainable CF of 5 in MF3). The maximum CF was determined by the experimental setup (minimum holdup, pumps). In all experiments, similar - non-optimized - process conditions based on experience were applied.

Table 3: Parameters applied for purification and concentration of Basolite A520

| Parameter | MF1 | MF2 | MF3 |
|---|---|---|---|
| Membrane unit | Unit 1 | Unit 1 | Unit 2 |
| Membrane | Atech 0.2 μm (MF 20n) | Atech 0.05 μm (UF 50A) | Atech 0.05 μm (UF 50A) |
| Module geometry | Single-channel, 6mm inner diameter, 1 m length (0.019 $m^2$) | | |
| Basolite A520 Batch | Batch 1 | Batch 1 | Batch 2 |
| Temperature (°C) | 40 | 40 | 60 |

(continued)

| Parameter | MF1 | MF2 | MF3 |
|---|---|---|---|
| TMP (bar) | 1 | 1 | 1*** |
| Cross-flow velocity (m/s) | 4 | | |
| Diafiltration factor (DF) | 8 | 8 | 9 |
| Concentration factor (CF) | 2.5* | 2.5* | 5** |
| * limited by minimum hold-up in membrane unit 1, ** limited by viscosity of the concentrated Basolite A520 slurry. *** the TMP was increased at a later stage of the experiment to increase permeate fluxes. | | | |

To evaluate the concentrations of $Na_2SO_4$ and Basolite A520, analytics of total solids content analysis using evaporation and sulfate elemental analysis at GMC for determination of $Na_2SO_4$ concentrations were applied. The main assumption in determining the concentrations of Basolite A520 is that the slurry only consists of $Na_2SO_4$, Basolite A520 and water. By that, the concentration of Basolite A520 can be calculated by the following formula:

$$c(\text{Basolite A520}) = c(\text{total solids}) - c(Na_2SO_4).$$

Thus; it is possible to calculate the retention of Basolite A520 by the membrane, given by the formula

$$R(\text{Basolite A520}) = 1\text{- } c(\text{Basolite A520,permeate}) / c(\text{Basolite A520,retentate})$$

indicating 100% retention in case no Basolite A520 is found in the permeate and 0% retention of both concentrations c(Basolite,A520,permeate) and c(Basolite,A520,retentate) are the same. The same calculations can be done for the retention of $Na_2SO_4$. Samples in both the retentate and the permeate were collected at defined DF and CF.

The MF experiments were evaluated by three criteria:

(1) Permeate flux as function of DF / CF.
(2) Retention of Basolite A520.
(3) $Na_2SO_4$ removing capability as function of DF (indicated by a decrease in conductivity and $Na_2SO_4$ concentration in the retentate loop).

Moreover, water flux measurements before and after the experiments were conducted at a TMP of 0.5 bar for evaluating the magnitude of membrane fouling effects. During the experiments, back flushing with permeate was applied at certain intervals to check its effect on membrane performance. As it was observed that in the end a lot of material was sticking to the walls (e.g. on the feed glass vessel), it was washed (cleaned) twice with VE water and both samples were analyzed for total solids concentration (which in the end was almost equal to the concentration of Basolite A520 as almost no salts were present).

3. Experimental results

In Figures 2 and 3, the MF permeate fluxes (left axes) and retentate conductivities (right axes) are shown as functions of the DF and CF respectively. Conductivity has been determined by measuring 100 mL of wash water with the standard conductivity measuring electrode.

The performance of both membranes (0.2 $\mu$m and 0.05 $\mu$m) was very similar. This indicates that, as expected, a boundary layer controlled permeation mechanism occurred. Both membranes reached very high permeate fluxes between 200 and 550 kg / $m^2$ h during the diafiltration step, which was then decreasing to values of about 50 kg/ $m^2$ h during concentration by a factor of approx. 2.5. Concerning conductivities, both membranes lead to final conductivities well below 500 $\mu$S/cm; with an extensive decline in the beginning of the experiments but a lower washing out effect at the end of the experiments (note the logarithmic scaling for conductivity). The decrease in conductivities during the course of the last DFs was very low which may either be explained by a blocking effect at elevated washing factors (very unlikely: also with a preconcentration by a factor of 2 in MF3, conductivities decreased significantly during diafiltration) or by the conductivity of the Basolite A520 itself.

The water flux measurements at TMP = 0.5 bar before and after the Basolite A520 filtration experiments, showed

the following: Water fluxes were 700 kg / $m^2$ h before and 1280 kg / $m^2$ h after the filtration in MF1, 574 kg / $m^2$ h and 555 kg / $m^2$ h in MF2, and 565 kg / $m^2$ h and 562 kg / $m^2$ h in MF3; thus indicating a very low magnitude of membrane fouling.

In detail,

Fig. 2 shows permeate fluxes and conductivities in the retentates as function of DF or CF in experiments 1 and 2. In both experiments, first a diafiltration followed by a concentration were conducted. During both experiments, the effect of back flushing on the permeate flux was tested. It was shown that this effect was minor or only visible for a small time interval (see spikes in the graphs).

Fig. 3 shows permeate fluxes and conductivities in the retentates as function of DF or CF in experiment 3. In this experiment, an initial concentration of the Basolite A520 slurry was followed by a diafiltration and by a final concentration. The conductivities shown are the ones during the diafiltration (a manual measurement device had to be used due to plugging of the online conductivity measurement device during the first concentration).

In Table 4, the resulting properties of the final retentates of the three experiments are summarized. As can be observed, the difference between retentate MF1 and MF2 was very low; each reaching a final Basolite A520 concentration of approx. 9.7 wt.-% and very low amounts of $Na_2SO_4$.

Table 4: Resulting Basolite A520 slurries after diafiltration and concentration

| Parameter | Final retentate MF1 | Final retentate MF2 | Final retentate MF3 |
|---|---|---|---|
| Total solid concentration (wt.-%) | 9.85 | 9.81 | 19.07 |
| Conductivity ($\mu$S/cm) | 413 | 291 | 391 |
| $Na_2SO_4$ concentration (wt.-%) | 0.12 | 0.15 | 0.15 |
| Basolite A520 concentration (wt.-%) | 9.73 | 9.66 | 18.85 |

5. Conclusion

The conducted experiments show, that it was possible to purify Basolite A520 slurries using DI water by means of membrane filtration. As can be seen in above tables, the concentration of $Na_2SO_4$ could be reduced form about 5.5 % by weight to values below 0.15 % by weight. The needed diafiltration volumes for that is 8 - 9 times the initial volume.

Furthermore, the experiments show that the concentration of Basolite A520 in the slurries could be increased from 4.5 % respectively 4.8 % by weight up to values of about 10 % by weight respectively about 19 % by weight.

C. Coating experiments

The aim of the slurry characterization and coating experiments was to show a general proof of principle and set the process in relation to other processing alternatives such as conventional spray drying and dispersing the filter cake.

1. Experimental

a) Materials:

Substrate

- Substrate 1: Nippon foil (1 N30) 120mmX20$\mu$m was used as substrates for peel test and film density measurements.

Three different substrates were used to test dip coating of complex geometries:

- Substrate 2: Segment of finned tube heat exchanger, aluminum plates with tube fitting, 390 g/$m^2$, fin pitch 2 mm.

- Substrate 3: Single finned tube heat exchanger plate, 311 g/$m^2$.

- Substrate 4: Polypropylene corrugated plastic, 287 g/$m^2$.

MOF-materials:

- Aluminum fumarate samples "Final Retentate of MF1 , 2 and 3" as described above, hereafter referred to as MF1-MOF, MF2-MOF and MF3-MOF
- conventionally filtrated and spray dried material (Basolite A520; BASF SE), hereafter referred to as SPD-MOF.
- Laboratory sample of dispersed filter cake ("WFC", Aluminum fumarate). hereafter referred to as WFC-MOF

Binder:

A water based polyacrylate binder (Joncryl 3030; BASF SE, $M_w$ > 200000) was used at a solid content of 19.6 wt.% with regard to the total dry mass of the coating.

Primer

Polyethyleneimine (Lupasol PS; BASF SE, about $M_w$ 750000) was used as primer. If not stated explicitly coatings were applied without primer layer.

b) Preparation of the Coating Compositions

Water was deposited into a polypropylene mixing container and SPD-MOF powder was added under manual stirring. Subsequently the binder dispersion was added and dispersed for 3 min at 2000 rpm using a centrifugal mixer (ARE310; Thinky inc).

The Polyethyleneimine solution (Lupasol PS; BASF SE) was diluted from its original solid content of 32.76 wt.% to 6.55 wt.% with demineralized water.

To give an indication whether the slurry is stable under process conditions, it was stirred by a magnetic rod in a 300 ml beaker at 100 rpm. Storage stability was tested by aging the slurry in a closed container, without stirring at ambient temperature (~20-25°C). In both cases viscosity was measured in time intervals after preparation.

c) Coating

The particular coating compositions were coated onto the substrate using a knife coating system (ZAA2300; ZUA2000; Zehntner GmbH) with a 200 μm gap setting at 150 mm/s and a deposition volume of 8 ml.

The primer solution (6.55wt.% Lupasol PS) was coated onto the substrate using a 25 μm wire bar applicator that was pulled over the substrate at 30 mm/s and a deposition volume of 2 ml.

d) Drying

The coating was dried by contact drying for 10 min at 60°C using an electrically temperature controlled vacuum plate (ACC188.230; Zehntner GmbH) and subsequently for >1h at 110°C in a drying cabinet (UN110Plus; Memmert).

e) Analytics:

Solid content was measured using a dry-weight-scale (setting 120°C; HB43-S; Mettler Toledo).

Viscosity was measured using a cone plate rheometer (MCR102, Anton Paar; PP50; 400μm gap; 25°C) at shear rates from 1 -/s to 1000 -/s.

Adhesion on a reference substrate was measured by a 90° peel test according to DIN 28510 with 50 mm/min pull rate, 25°C at a relative humidity of -40%rH.

Density was calculated from a measurement of coating weight (Excellence Plus, $10^{-4}$g; Mettler Toledo) and thickness (ID-H0530, $10^{-4}$ mm; Mitutoyo Corp) on a 50 $cm^2$ film sample. The sample was cut out of a coated aluminum substrate using a circular knife cutter (150805; Karl Schroder KG).

f) Nomenclature:

A dispersion of Basolite A520 and water (as prepared by membrane filtration or dispersion of SPD-MOF powder

or WFC-MOF in water) will be referred to as "MOF premix". After binder addition and dilution, the final coating formulation will be referred to as "MOF/binder slurry".

Properties are determined by the processing route but can be tuned by changing process parameters as well (i.e. feed rate during crystallization, temperature during spray drying). Whether the changes observed here are due to processing route only or may be optimized has not been checked in detail and is outside the scope of this study.

2. Properties of the Coating Compositions

a) Viscosity

Ideally, coating compositions should have a low viscosity at medium to high shear-rates (100-1000 -/s), a high viscosity at low shear rates < 10 /s, a high yield point, and should be stable under process and storage conditions.

With respect to a coating application, both the fluid dynamic properties of the coating formulation as well as the properties of the dried film need to be considered. The results presented here give an indication whether the material produced with the membrane filtration process described above is suitable for a coating application but does not represent a thorough characterization for coating process development.

A coating composition is composed of a solvent (here water), active material (here Aluminum Fumarate (A520)), a binder (here Joncryl 3030) and processing additives. To keep the comparison as simple as possible, binder type, binder content and as far as possible solid content were kept constant. No processing additives were used.

Ideally, coating composition should exhibit a low viscosity at medium to high shear-rates (100-1000 -/s), a high viscosity at low shear rates < 10 /s, a high yield point, and should be stable under process and storage conditions. Rheological yield point was not measured explicitly but may be estimated to be proportional to zero shear viscosity for similar compositions.

For a dip coating process, viscosity is limited by two characteristic boundaries. Too high viscosity will lead to blocking due to incomplete draining of the fluid from the coated structure. Too low viscosity will lead to inhomogeneous coating as the fluid forms rivulets running down the substrate. A dip coating process aiming for high coating weights will typically operate close to the upper viscosity limit since this allows for a higher solid content (higher solid content implies that higher dry film thicknesses can be realized and less water needs to be evaporated.) Viscosity may change under process or storage conditions. A change in viscosity under process conditions may indicate destabilization, structure formation or incomplete dispersion and is not acceptable in a technical coating process.

Table 5. Comparison of viscosity flow curve of Aluminium fumerate prepared by the membrane filtration process (MF1-MOF) and spray drying (SD6-MOF) process. Measurement was done at 25°C and a solid content of 7.5wt.-%.

| Material | Shear rate [1/s] | Viscosity [Pas] |
|---|---|---|
| MF1-MOF | 1 | 1 |
| MF1-MOF | 2 | 0,9 |
| MF1-MOF | 5 | 1 |
| MF1-MOF | 10 | 0,7 |
| MF1-MOF | 20 | 0,5 |
| MF1-MOF | 50 | 0,2 |
| MF1-MOF | 100 | 0,09 |
| MF1-MOF | 200 | 0,04 |
| MF1-MOF | 500 | 0,02 |
| MF1-MOF | 1000 | 0,013 |
| SD6-MOF | 1 | 0,2 |
| SD6-MOF | 2 | 0,2 |
| SD6-MOF | 5 | 0,2 |
| SD6-MOF | 10 | 0,1 |
| SD6-MOF | 20 | 0,05 |
| SD6-MOF | 50 | 0,025 |

(continued)

| Material | Shear rate [1/s] | Viscosity [Pas] |
|---|---|---|
| SD6-MOF | 100 | 0,013 |
| SD6-MOF | 200 | 0,009 |
| SD6-MOF | 500 | 0,006 |
| SD6-MOF | 1000 | 0,005 |

A reliable measurement of a premix from re-dispersed WFC-MOF was not possible because the slurry became too low viscous and subsequently unstable during dilution to 7.5 % by weight.

b) Process stability

To evaluate process stability, the MOF premix from MF3-MOF and the MOF/binder slurry (MF3-MOF with binder) were measured at different time intervals after end of membrane filtration. No change in viscosity could be observed within the first 5 hours. The slurry can thus be assumed to be stable for process relevant timespans.

After binder addition no change in viscosity was observed within process relevant time spans as long as the dispersion was continually stirred, as well.

3. Results and discussion

Since the purification and concentration method can be expected to change particle morphology, it is important to check the impact on film properties. Accordingly, mechanical properties such as cracking, film density and adhesion were evaluated.

a) Crack formation

Shrinkage during drying can cause cracking of a coated film. The tendency of a film to crack depends primarily on composition (e.g. binder type and content, solvent type and surface tension), particle size distribution and morphology and film thickness. Here composition, drying temperature and film thickness were kept constant to show the impact of particle size and morphology.

Crack formation of three different dried coating compositions, comprising MOF (washed MOF-WFC, Membrane filtrated MF1-MOF and SPD-MOF) were compared.

It could be observed, that the amount of cracking increased dorm almost none for the WFC-MOF, slight crack formation form the membrane-filtrated MF1-MOF and severe for the SPD-MOF.

The MF1-MOF thus shows more cracking than the MOF-WFC material but less than the SPD-MOF. The critical cracking thickness for this composition was determined for wet filter cake material to be 120 μm. While cracking can be suppressed e.g. by lower coating thickness or higher binder content, the spray dried material used in this study can be seen as unsuitable for a high capacity coating application.

b) Film Density

The density of a coated film is an important parameter for a sorption heat pump coating. As coating thickness will be limited by module geometry and required minimal adhesion for a given composition, density is proportional to the power density of the overall system.

Table 5: Film density of Aluminum-Fumarate films prepared by different processes

| Material | Density [g/cm$^3$] |
|---|---|
| SPD-MOF | 0.34 |
| WFC-MOF* | 0.42 |

(continued)

| Material | Density [g/cm$^3$] |
|---|---|
| MF1-MOF und MF3-MOF** | 0.59 |
| * Some air entrainment defects were observed for the WFC-MOF and the actual bulk density may be slightly higher ** The value for membrane filtration is an average of coatings produced from MF1-MOF and MF3-MOF material with less than 5% relative deviation. | |

c) Adhesion

The adhesion of a coating depends primarily on composition, drying conditions, film thickness, particle size distribution and morphology, and surface properties. Here, composition and drying conditions were kept constant. Since adhesion cannot be measured reproducibly on complex surface of a heat exchanger fin, measurements were conducted on aluminum foil (Nippon) as reference substrate using a standardized 90°-peel test (see experimental section for details). The required minimal adhesion depends on the operating conditions in the final application but values of less than 5 N/m can be considered insufficient for most technical applications.
Adhesion of MOF coatings from material produced in experiment 3 by membrane filtration and by re-dispersing filter cake were evaluated.

Table 6: Film adhesion as measured by a 90° peel test as a function of film thickness

| Material | Film thickness [μm] | Adhesion [N/m] |
|---|---|---|
| WFC-MOF | 30 | 25 |
| WFC-MOF | 81 | 14 |
| MF3-MOF | 61 | 43 |
| MF3-MOF | 93 | 24 |
| MF3-MOF | 115 | 17 |
| MF3-MOF | 192 | 14 |

As expected the adhesion decreases with coating weight (corresponds to film thickness).
Adhesion of the MOF-MF3-coatings is lower compared to the WFC MF3-MOF-coatings. However, all measured values are higher than 10 N/m indicating that a binder content of 19.6 wt.-% is sufficient for most applications.
For SPD-MOF prepared by spray drying and MF-MOF prepared by membrane filtration, an increase in adhesion was observed when a polyethyleneimine primer layer has been applied to the substrates surface (PCT/EP2017/071096).This effect was reproduced with the membrane filtrated material MF3-MOF as summarized in Table 8.

Table 7: Impact of a polyethyleneimine primer coating and a water bath test

| | Adhesion [N/m] |
|---|---|
| No primer, as prepared | 14 |
| No primer, after water bath test | 11 |
| With primer, as prepared | 24 |
| With primer, after water bath test | 23.5 |

Even though the primer is water based, adhesion remains high after immersion in boiling water for 3 min (denoted as "water bath test").The water bath test emulates the harsh operating conditions in a sorption heat pump by immersing the coating in boiling water for 3 min.

d) Dip coating of complex geometries

Depending on the application, MOF-coatings need to be applied onto different substrates. Substrates may

differ with respect to surface material and geometry. Complex geometries may lead to highly inhomogeneous coating weight as fluid can accumulate in recesses or completely block narrow gaps in a tube finned heat exchanger. Polymeric surface may lead to de-wetting or low adhesion. The viscosity of the MOF-coating compositions needs to be adapted to the target geometry. Too high viscosity will lead to blocking or too high coating weights while too low viscosity may cause rivulet formation and very low coating weights.

The substrates were hand dipped, dried at room temperature for 30 minutes and afterwards at 110°C for more than 1 hour. Three exemplary geometries were coated with Basolite A520 MOF-MF1 mixed with polyacrylic binder and diluted with water to a suitable solid content. The binder mass fraction in solid was kept constant at 19.6 % by weight, no processing additives were used and the solid content was varied between 12.5 to 15 5 by weight to adapt viscosity.

- Substrate 2: Segment of finned tube heat exchanger, aluminum plates with tube fitting, 390 g/$m^2$, fin pitch 2 mm, 12,5 % by weight
- Substrate 3: Single finned tube heat exchanger plate, 311 g/$m^2$, solid content (MOF) 15 % by weight
- Substrate 4: Polypropylene corrugated plastic, 287 g/$m^2$, 12,5 % by weight

Though some cracking can be observed for the highly corrugated polypropylene substrate, all coatings show good adhesion and only minor visible defects.

4. Conclusion

In summary, the MF-MOF-containing coating compositions according to the invention show several improved properties over coating compositions comprising SPD-MOF or WFC-MOF.

In particular, the coating compositions according to the invention show a reproducible high density of the MOF-layer in combination with sufficient high critical cracking thickness. No significant differences in the properties of coating compositions or MOF-films could be observed between membrane filtration experiments MF1, MF2 and MF3. A compact overview of the results is given in Table 6. Moreover, the energy consumption is significant lower for the inventive coating compositions.

Table 8: Estimate values for important process, slurry and film properties

| | Process energy consumption (number of drying steps) | Film Density [g/$cm^3$] | Adhesion (90° peel test @80 μm [N/m] | Crack formation (critical cracking thickness, μm) | Slurry viscosity [Pas], 7.5 % by weight at 10/s |
|---|---|---|---|---|---|
| SPD-MOF | (3) | 0.33 | 21 | ~20 | 0.1 |
| WFC-MOF | (2) | 0.41 | 34 | ~120 | <0.04 |
| MF1-MF3 | (1) | 0.6 | 14 | ~80 | 0.6 |

## Claims

1. Process for conditioning a raw suspension SR comprising at least one metal-organic framework and at least one suspension medium SM1,
wherein the at least one metal-organic framework comprises at least one at least bidentate organic compound coordinated to at least one metal ion,
wherein the raw suspension SR is conditioned by means of at least one membrane filtration, to obtain a product suspension SP, comprising the at least one metal-organic framework and at least one suspension medium SM2.

2. The process according to claim 1, wherein the at least one membrane filtration comprises at least one purification step, or at least concentration step, or at least one purification step and at least one concentration step.

3. The process according to claim 1 or 2, wherein the at least one membrane filtration comprises at least one purification step and at least concentration step.

4. The process according to claim 2 to 3, wherein the at least one purification step and/or the at least one concentration step is carried out as diafiltration.

5. The process according to claim 4, wherein the diafiltration medium is selected from the group consisting of water, methanol, ethanol, i-propanol, n-butanol, i-butanol, sec-butanol or a mixture of two or more thereof, preferably water.

6. The process according to any one of claims 1 to 5, wherein the separation layers of the membrane has a pore size in the range from 50 to 800 nm, preferably from 50 to 200 nm.

7. The process according to any one of claims 1 to 6, wherein suspension medium SM1 and suspension medium SM2 are independently from each other selected from water, methanol, ethanol, i-propanol, n-butanol, i-butanol, sec-butanol or a mixture of two or more thereof.

8. The process according to any one of claims 1 to 7, wherein suspension medium SM1 and suspension medium SM2 are equal, preferably water.

9. The process according to any one of claims 1 to 8, wherein the raw suspension SR is obtained by a method for the preparation of the at least one metal-organic framework, comprising

- reacting at least one metal salt with at least one at least bidentate organic compound, preferably in the presence of at least one additional compound, in a reaction medium, wherein the reaction medium corresponds to the at least one suspension medium SM1.

10. The process according to any one of claims 1 to 9, wherein the at least one metal ion is an ion of Zn, Al, Mg, Cu, Mn, Fe, Co, Ni, Ti, Zr, Y, Sc, V, In, Ca, Cr, Mo, W, Ln, preferably Cu, Zn, Al, Mg, Zr and Fe.

11. The process according to any one of claims 1 to 10, wherein the at least one metal ion is an ion of Cu, Zn, Mg or Al.

12. The process according to any one of claims 1 to 11, wherein the at least one at least bidentate organic compound is derived from a di-, tri- or tetracarboxylic acid or a monocyclic, bicyclic or polycyclic ring system which is derived from at least one heterocycle selected from the group consisting of pyrrole, alpha-pyridone and gamma-pyridone.

13. The process according to any one of claims 1 to 12, wherein the at least one at least bidentate organic compound is a di-, or tricarboxylic acid or substituted or unsubstituted imidazole.

14. The process according to any one of claims 1 to 13, wherein the metal-organic framework is Cu-1,3,5-benzenetricarboxylate, Zn-2-methylimidazolate, Al-fumarate, Al-terephthalate or Mg-formate, preferably Al-fumarate.

15. Method for coating at least part of a surface of a substrate with an active layer, comprising at least one metal-organic framework, comprising

a) bringing at least part of the surface of the substrate into contact with a coating composition, wherein the coating composition comprises the product suspension according to any one of claims 1 to 14, comprising at least one metal-organic framework and at least one binder.

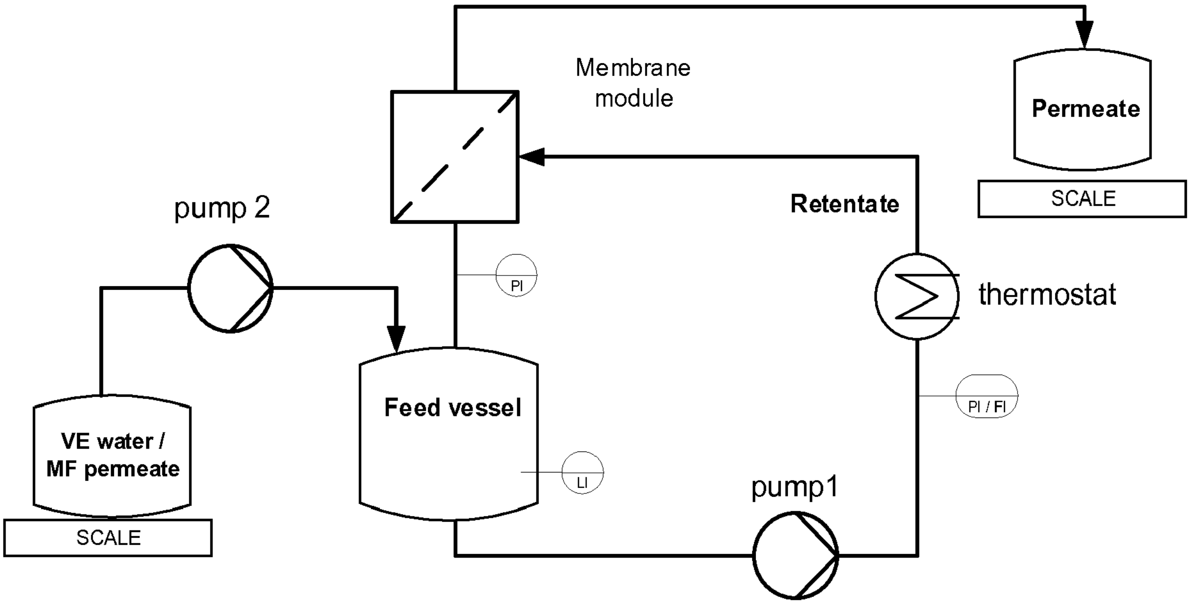
Membrane
module
Permeate
SCALE
Retentate
pump 2
PI
thermostat
PI / FI
Feed vessel
VE water /
MF permeate
LI
pump1
SCALE

Fig. 1

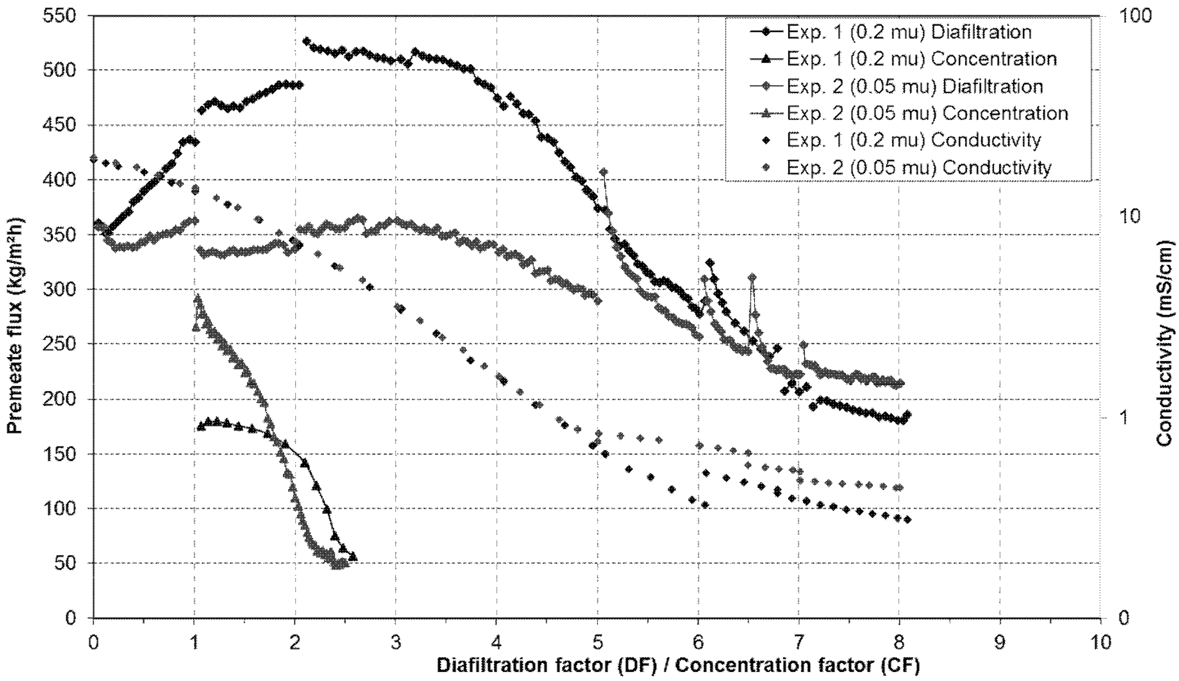
Exp. 1 (0.2 mu) Diafiltration
Exp. 1 (0.2 mu) Concentration
Exp. 2 (0.05 mu) Diafiltration
Exp. 2 (0.05 mu) Concentration
Exp. 1 (0.2 mu) Conductivity
Exp. 2 (0.05 mu) Conductivity
Premeate flux (Kg/m²h)
Conductivity (mS/cm)
Diafiltration factor (DF) / Concentration factor (CF)
0
50
100
150
200
250
300
350
400
450
500
550
0
1
10
100
0
1
2
3
4
5
6
7
8
9
10

Fig. 2

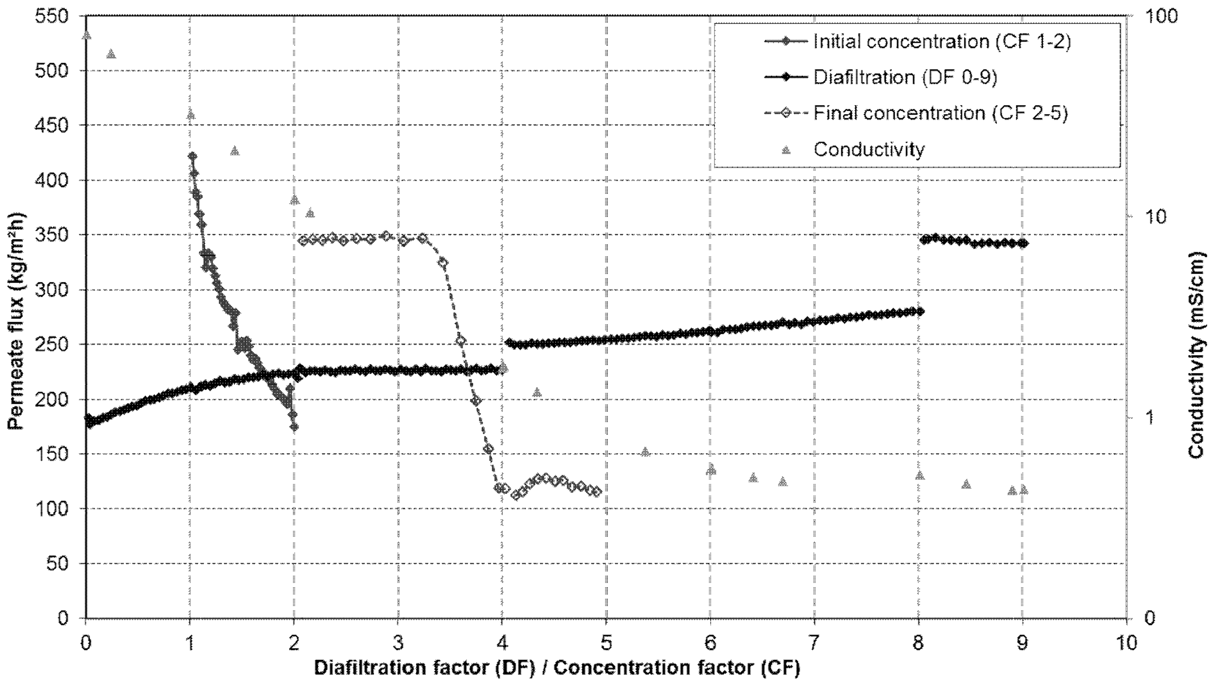
550
500
450
400
350
300
250
200
150
100
50
0
Permeate flux (kg/m²h)
100
10
1
0
Conductivity (mS/cm)
0 1 2 3 4 5 6 7 8 9 10
Diafiltration factor (DF) / Concentration factor (CF)
Initial concentration (CF 1-2)
Diafiltration (DF 0-9)
Final concentration (CF 2-5)
Conductivity

Fig. 3

Europäisches Patentamt
European Patent Office
Office européen des brevets

# EUROPEAN SEARCH REPORT

Application Number
EP 18 20 6469

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/154222 A1 (MULLER ULRICH [DE] ET AL) 14 July 2005 (2005-07-14)<br>* paragraphs [0001], [0017], [0021], [0052], [0075], [0105], [0107], [0114] *<br>----- | 1-15 | INV.<br>B01D61/14 |
| A | US 2012/082864 A1 (LEUNG EMI [US] ET AL) 5 April 2012 (2012-04-05)<br>* examples 1,2 *<br>----- | 1-15 | |
| X,D | WO 2018/036997 A1 (BASF SE [DE]) 1 March 2018 (2018-03-01) | 15 | |
| A | * claims 1,2,18; example 2 *<br>----- | 1-14 | |
| A | US 2016/214080 A1 (MATOGA DARIUSZ [PL]) 28 July 2016 (2016-07-28)<br>* paragraph [0021] *<br>----- | 1-15 | |

TECHNICAL FIELDS SEARCHED (IPC)

B01D
C07C
C07F
G01N
B01J

The present search report has been drawn up for all claims

1

| Place of search | Date of completion of the search | Examiner |
|---|---|---|
| Munich | 15 May 2019 | Hennebrüder, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 20 6469

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2019

| Patent document cited in search report | Publication date | Patent family member(s) | Publication date |
|---|---|---|---|
| US 2005154222 A1 | 14-07-2005 | AT 370956 T | 15-09-2007 |
| | | BR PI0506420 A | 26-12-2006 |
| | | CN 1910191 A | 07-02-2007 |
| | | DE 602005002130 T2 | 13-12-2007 |
| | | EP 1716157 A1 | 02-11-2006 |
| | | ES 2290879 T3 | 16-02-2008 |
| | | JP 2007534658 A | 29-11-2007 |
| | | KR 20060110336 A | 24-10-2006 |
| | | US 2005154222 A1 | 14-07-2005 |
| | | WO 2005068474 A1 | 28-07-2005 |
| | | ZA 200605687 B | 28-11-2007 |
| US 2012082864 A1 | 05-04-2012 | NONE | |
| WO 2018036997 A1 | 01-03-2018 | CN 109642094 A | 16-04-2019 |
| | | KR 20190043570 A | 26-04-2019 |
| | | WO 2018036997 A1 | 01-03-2018 |
| US 2016214080 A1 | 28-07-2016 | EP 3041823 A2 | 13-07-2016 |
| | | PL 230327 B1 | 31-10-2018 |
| | | US 2016214080 A1 | 28-07-2016 |
| | | WO 2015030617 A2 | 05-03-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009115513 A1
- WO 2010012715 A1
- WO 200909277 A
- WO 2018036997 A1
- EP 2017071096 W


### Non-patent literature cited in the description


- **M. TATLIER et al.** *Microporous and Mesoporous Materials,* 2000, vol. 34, 23-30
- *Pure & Applied Chem.,* 1983, vol. 57, 603-619